# EUROPEAN PATENT APPLICATION

(11) **EP 4 806 930 A1**
(43) Date of publication of application: **16.09.2026**
(21) Application number: 24902920.8
(22) Date of filing: 12.12.2024
(51) Int. Cl.: C08F 283/06, C07D 317/42, C07F 7/18, C08F 216/12, C08F 2/44, H01M 10/0565, H01M 10/0525, H01M 10/054

(54) **PREPARATION METHOD FOR FLUORO-1,3-DIOXOLANE COMPOUND, IN-SITU SOLID ELECTROLYTE, AND PREPARATION METHOD THEREFOR AND USE THEREOF**

(30) Priority: 12.12.2023 CN 202311696238
(71) Applicant: Liongo (Changzhou) New Energy Co., Ltd., Changzhou, Jiangsu 213300 (CN)
(72) Inventor: XIE, Ping, hangzhou, Jiangsu 213300 (CN); LI, Peize, hangzhou, Jiangsu 213300 (CN); HUANG, Jian, hangzhou, Jiangsu 213300 (CN); ZHOU, Longjie, hangzhou, Jiangsu 213300 (CN); LI, Lifei, hangzhou, Jiangsu 213300 (CN); WANG, Shengyue, hangzhou, Jiangsu 213300 (CN)
(74) Representative: Comoglio, Elena
(86) International application number: PCT/CN2024/138898
(87) International publication number: WO 2025/124503

(57) **Abstract**

The present invention relates to the technical field of solid-state batteries, and provides a preparation method for a fluoro-1,3-dioxolane compound, an in-situ solid electrolyte, and a preparation method therefor and a use thereof. The in-situ solid electrolyte is obtained by curing a mixture comprising the fluoro-1,3-dioxolane compound, a free radical polymerization monomer, a first initiator, a second initiator, an electrolyte salt, and a non-aqueous organic solvent. By means of the exothermic ionic polymerization of fluoro-1,3-dioxolane compound at room temperature, free radical monomer polymerization is initiated, avoiding non-uniform polymerization caused by external heating. In addition, the free radical monomer polymerization can consume heat released by ion polymerization, resulting in a milder curing process without bubble residues, so that the in-situ solid electrolyte is more uniform in polymerization, and has better contact with an electrode. Upon research, the in-situ solid electrolyte has a wide electrochemical window, an oxidation voltage greater than 4.5 V, and ionic conductivity greater than 5×10⁻⁴ S/cm.

## Description

### CROSS-REFERENCE TO RELATED APPLICATION

This application claims priority to Chinese Patent Application No. 2023116962388 filed with China National Intellectual Property Administration on December 12, 2023 and entitled "PREPARATION METHOD FOR FLUORO-1,3-DIOXOLANE COMPOUND, IN-SITU SOLID ELECTROLYTE, AND PREPARATION METHOD THEREFOR AND USE THEREOF", the entire content of which is incorporated into this application by reference.

### TECHNICAL FIELD

The present invention belongs to the technical field of solid-state batteries, and specifically relates to a preparation method for a fluoro-1,3-dioxolane compound, an in-situ solid electrolyte, and a preparation method therefor and a use thereof.

### BACKGROUND ART

Solid electrolyte is one of the important development directions in electric vehicle battery technologies, and is closely related to electric vehicle safety. In recent years, a variety of solid electrolytes have been developed, including inorganic solid electrolytes (such as sulfides, oxides, halides and anti-perovskites), and polymer electrolytes. Among them, inorganic solid electrolytes have high ionic conductivity, but exhibit poor chemical stability or mechanical properties. Polymer electrolytes possess good mechanical properties, but have poor ionic conductivity. Furthermore, since complex preparation processes of conventional solid electrolytes, researchers have proposed an in-situ solidification technology for preparing in-situ solid electrolytes, in which electrolyte materials are blended with electrolyte solution and then polymerized at low temperature, thereby forming a stable in-situ solid electrolyte. The in-situ solid electrolytes have excellent thermal stability, electrochemical performance and safety, can effectively improve safety and reliability of batteries, and achieve high energy density at low costs.

At present, common polymerization monomers for in-situ solid electrolytes mainly include acrylates, epoxy compounds, substances containing olefinic bonds and other substances. Among them, 1,3-dioxolane (DOL), as an epoxy compound, has been widely studied due to good ionic conductivity, low interface resistance, high mechanical strength and good lithium metal compatibility after polymerization. However, DOL usually exhibits low polymerization degree during the in-situ polymerization in batteries, forming gel-like electrolytes that result in a low oxidative voltage window, limiting its application in high-voltage batteries.

To improve the oxidation window of the in-situ solid electrolyte, researchers have proposed introducing strongly electronegative groups, such as halogen groups, haloalkyl groups, or thioalkyl groups, into the structure of the polymerization monomer. Patent CN107353276A discloses a method for preparing halo-2,2-di(perfluoro substituent)-1,3-dioxolane. Under the action of a catalyst, 2,2-di(perfluoro substituent)-1,3-dioxolane reacts with Cl₂ and HF to obtain halo-2,2-di(perfluoro substituent)-1,3-dioxolane. However, the substances synthesized in this patent are mainly halo-2,2-di(perfluorosubstituted)-1,3-dioxapentane, with low purity.

Furthermore, in the process of preparing large-size solid electrolytes through in-situ polymerization, due to the different thermal conductivity of different phases (solid phase, liquid phase, etc.), the precursor solution components of the solid electrolyte near the electrode edges are solidified upon heating earlier than the internal components, leading to disparities in polymerization molecular weight, solidification degree and other properties at different positions. Consequently, the resulting solid electrolyte fails to achieve satisfactory uniformity, which exerts adverse effects on battery performance. Against this background, Patent CN114122512A discloses a method involving copolymerization of room-temperature polymerization monomers and high-temperature polymerization monomers. The framework network formed by room-temperature curable monomers confines high-temperature polymerization monomers within it, thus alleviating the adverse effects of uneven heating and non-uniform heat conduction of heating equipment on polymerization at high temperatures. This method can improve polymerization uniformity to a certain extent, but the subsequent high-temperature curing process still suffers from non-uniform heating. In addition, polymerization is an exothermic reaction that causes vaporization of small organic molecules. The polymerization product usually has high viscosity, making it difficult for the vaporized small molecules to escape. The trapped small molecular gases form microbubbles in the electrolyte. These bubbles cannot conduct ion transport, hinder ion migration in the electrolyte, and even cause partial active materials to lose contact with the electrolyte, resulting in battery capacity decay. Furthermore, the vaporized small molecules from the electrolyte have a low voltage window and low ignition point, and may decompose and support combustion during practical applications, posing potential safety hazards.

### SUMMARY

The purpose of the present invention is to provide a preparation method for a fluoro-1,3-dioxolane compound, an in-situ solid electrolyte, and a preparation method therefor and use thereof. The in-situ solid electrolyte has high ionic conductivity and a wide electrochemical window, and can be applied to high-voltage systems.

To achieve the above purposes of the present invention, the following technical solutions are adopted.

In a first aspect, the present invention provides an in-situ solid electrolyte, which is obtained by curing a mixture, wherein
the mixture comprises a fluoro-1,3-dioxolane compound, a free radical polymerization monomer, a first initiator, a second initiator, an electrolyte salt and a non-aqueous organic solvent;
the fluoro-1,3-dioxolane compound is any one selected from the group consisting of Formulas I to X below:

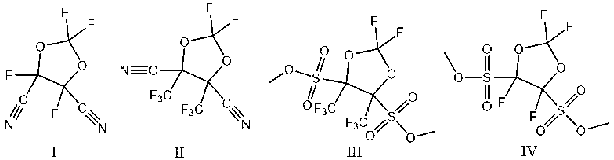

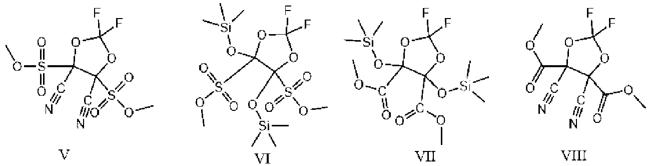

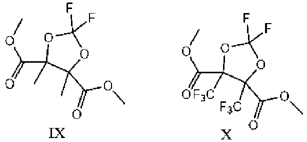

Further, the free radical polymerization monomer is any one or more selected from the group consisting of vinyl ethylene carbonate, methyl vinyl sulfone, ethyl vinyl sulfone, triethylene glycol divinyl ether, methyl methacrylate, vinyl acetate, 1,3-propene sultone, acrylamide, ethyl methacrylate, n-butyl methacrylate, vinylene carbonate, maleic anhydride, succinonitrile, trimethylolpropane triacrylate, ethylene glycol dimethacrylate, vinyl acetate, polyethylene glycol diacrylate and n-butyl acrylate.

Further, the first initiator is selected from azo initiators and/or peroxide initiators.

Further, the second initiator is any one or more selected from the group consisting of lithium hexafluorophosphate, lithium tetrafluoroborate, lithium difluoro(oxalato)borate, sodium hexafluorophosphate, sodium tetrafluoroborate, sodium difluoro(oxalato)borate, boron trifluoride, niobium pentachloride, titanium tetrachloride, aluminum chloride, ferric chloride and aluminum trifluoromethanesulfonate.

Further, the electrolyte salt comprises lithium salt and sodium salt.

Further, the lithium salt is any one or more selected from the group consisting of LiPF₆, LiClO₄, LiBF₄, LiPOF₂, LiTFSI, LiFSI, LiODFB and LiBOB.

Further, the sodium salt is any one or more selected from the group consisting of NaPF₆, NaClO₄, NaBF₄, NaTFSI, NaFSI and NaODFB.

Further, the non-aqueous organic solvent is any one or more selected from the group consisting of ethylene carbonate, propylene carbonate, diethyl carbonate, ethyl methyl carbonate, dimethyl carbonate and ethylene glycol dimethyl ether.

Further, the in-situ solid electrolyte has an oxidation voltage greater than 4.5 V, suitable for high-voltage systems, and an ionic conductivity greater than 5×10⁻⁴ S/cm.

In a second aspect, the present invention provides a preparation method for an in-situ solid electrolyte involved in the above technical solutions, including the following steps:
S1: mixing a first initiator and a fluoro-1,3-dioxolane compound shown in any one of Formulas I to X to obtain a first polymerization precursor solution; mixing an electrolyte salt, a second initiator, a free radical polymerization monomer and a non-aqueous organic solvent to obtain a second polymerization precursor solution;
S2: mixing the first polymerization precursor solution and the second polymerization precursor solution to obtain a polymerization precursor electrolyte, injecting the polymerization precursor electrolyte into a battery containing a positive electrode active material for curing to obtain the in-situ solid electrolyte.

Further, in step S1, the mass ratio of the non-aqueous organic solvent to the free radical polymerization monomer is 5: (1~10).

Further, the mass fraction of the first initiator in the first polymerization precursor solution is 0.01%~5%.

Further, the mass fraction of the second initiator in the second polymerization precursor solution is 0.01%~3%.

Further, the mass fraction of the electrolyte salt in the second polymerization precursor solution is 12%~30%.

Further, in step S2, the mass ratio of the first polymerization precursor solution to the second polymerization precursor solution is 1: (1~5).

Further, the polymerization is room-temperature polymerization, and the duration of the polymerization is 12~72 h.

In a third aspect, the present invention provides a solid-state secondary battery, including a positive electrode, a negative electrode, a separator and an electrolyte;
the electrolyte is the in-situ solid electrolyte involved in the above technical solutions.

Further, the solid-state secondary battery is a solid-state lithium-ion battery or a solid-state sodium-ion battery.

In a fourth aspect, the present invention provides a preparation method for a fluoro-1,3-dioxolane compound, including the following steps:
mixing a vinyl compound solution and CO₂F₂ under a protective atmosphere, allowing an addition reaction to proceed under the catalysis of a foamed metal, removing the solvent, and purifying the product to obtain the fluoro-1,3-dioxolane compound.

Further, the protective atmosphere is argon.

Further, the foamed metal is any one or more selected from the group consisting of foamed iron, foamed nickel and foamed copper.

Further, the molar ratio of the vinyl compound in the vinyl compound solution to CO₂F₂ is 1: (1~5).

Further, the temperature of the addition reaction is -50 °C~-25 °C, and the duration of the addition reaction is 6~48 h.

Further, the vinyl compound solution is obtained by mixing a vinyl compound and a solvent at -50 °C~-25 °C under a protective atmosphere, followed by heating to room temperature.

Further, the solvent in the vinyl compound solution is any one or more selected from the group consisting of acetonitrile, dichloromethane, trichloromethane, dichloroethane, propanol, acetone, dioxane, tetrahydrofuran, methyl ethyl ketone, n-butanol, ethyl acetate, diethyl ether and chloroform.

Further, the vinyl compound in the vinyl compound solution is any one selected from the group consisting of Formula A to Formula J:

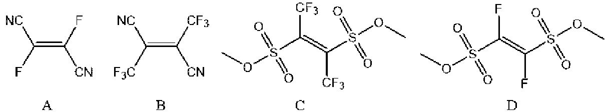

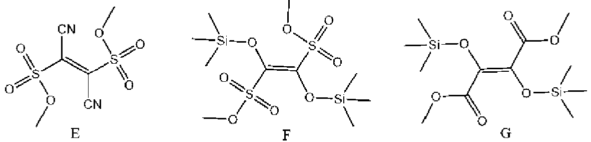

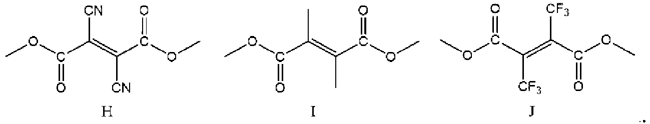

Further, the molar ratio of the vinyl compound to the solvent is 1: (2~10).

Further, the heating rate is 0.1~5 °C/min.

Further, after the addition reaction is completed, the resultant is heated to room temperature under a protective atmosphere, and then the solvent is removed to obtain the fluoro-1,3-dioxolane compound.

In the present invention, the room temperature refers to a temperature of 20 °C~30 °C, preferably 25 °C.

### DETAILED DESCRIPTION OF EMBODIMENTS

In view of the problems of low oxidation voltage and low ionic conductivity of in-situ solid electrolytes in existing art, the present invention firstly provides a polymerization monomer for in-situ solid electrolytes, namely fluoro-1,3-dioxolane compound (FDOL), the preparation method of which includes the following steps:

carrying out an addition reaction between a vinyl compound solution and CO₂F₂ at - 50 °C~-25 °C under a protective atmosphere in the presence of a foamed metal, removing the solvent, and purifying the product to obtain FDOL.

In the present invention, the vinyl compound solution is obtained by mixing a vinyl compound and a solvent at a low temperature of -50 °C~-25 °C, preferably -45 °C~-30 °C, under a protective atmosphere, and then heating the mixture to room temperature. The above low temperature is controlled by liquid nitrogen. The solvent is an inert solvent, and may specifically be any one or more selected from acetonitrile, dichloromethane, trichloromethane, dichloroethane, propanol, acetone, dioxane, tetrahydrofuran, methyl ethyl ketone, n-butanol, ethyl acetate, diethyl ether and chloroform.

The present invention imposes no particular limitation on the source of the aforementioned solvent, and commercially available products are generally acceptable.

The protective atmosphere is one of high-purity argon and nitrogen, preferably high-purity argon.

The vinyl compound is any one selected from Formula A to Formula J:

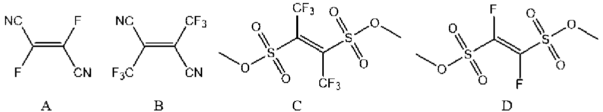

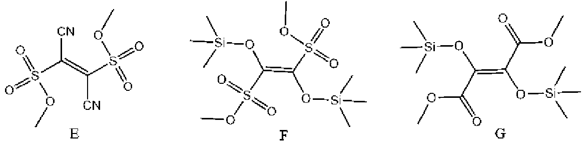

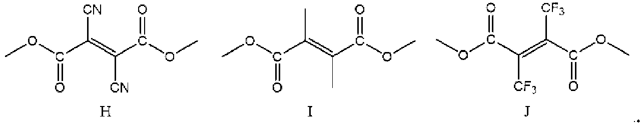

In some embodiments of the present invention, the molar ratio of the vinyl compound to the solvent is 1: (2~10), and may specifically be 1:2, 1:3, 1:4, 1:5, 1:6, 1:7, 1:8, 1:9 or 1:10. As the reaction yield is sensitive to the heating rate, the heating rate used in the present invention is 0.1 °C/min~5 °C/min, and may specifically be 0.1 °C/min, 0.5 °C/min, 1 °C/min, 2 °C/min, 3 °C/min, 4 °C/min or 5 °C/min, etc.

After obtaining the vinyl compound solution, according to the present invention, the vinyl compound solution and CO₂F₂ undergo an addition reaction at -50 °C~-25 °C, preferably at -45 °C~-30 °C, in the presence of a foamed metal under a protective atmosphere. The reaction equation is shown as follows: 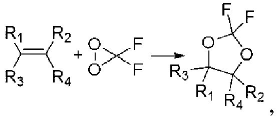 wherein R₁, R₂, R₃ and R₄ are each independently one or more selected from H, F, sulfonic acid group, carboxylate group, trimethylmethoxysilane and cyano group, provided that they are not all H simultaneously nor all F simultaneously. Among them, F element can further improve the oxidative stability of the electrolyte; sulfonic acid group can enhance the performance of batteries in gas evolution inhibition and high-temperature resistance; carboxylate group itself has good stability and a strong ability to complex lithium/sodium ions, effectively improving the low-temperature performance of batteries; trimethylmethoxysilane and cyano functional groups can remove excess HF generated in the electrolyte, suppressing battery performance degradation caused by high HF content and excessive gas production.

The protective atmosphere is as described above and will not be repeated herein.

The foamed metal, acting as a catalyst for the addition reaction of the vinyl compound with CO₂F₂, is predominantly foamed transition metal, and may specifically be any one or more selected from foamed iron, foamed nickel and foamed copper.

In the present invention, the molar ratio of the vinyl compound in the vinyl compound solution to CO₂F₂ is 1: (1~5), preferably 1:1.

In some embodiments of the present invention, liquid nitrogen is used to control the temperature at -50 °C~-25 °C. The vinyl compound solution is added into a stainless-steel passivation reactor, CO₂F₂ is condensed into the reactor, and the foamed metal is introduced to catalyze the reaction. At the low temperature of -50 °C~-25 °C, the vinyl compound and CO₂F₂ undergo an addition reaction on the surface of the foamed metal for 6 h~48 h, preferably for 12 h~30 h.

In some embodiments of the present invention, after the addition reaction is completed, the solvent is removed to obtain pure FDOL.

Under protective atmosphere, the heating rate is controlled to allow the temperature of the reactor to reach room temperature within 12 h~48 h, preferably within 20 h~30 h, so as to facilitate subsequent solvent removal by rotary evaporation to obtain pure FDOL.

In the present invention, the rotary evaporation is performed according to conventional technical means well known by those skilled in the art at a temperature of 35 °C~60 °C, preferably 40 °C.

In the present invention, the aforementioned product FDOL exhibits a yield above 90% and can be separated and purified via column chromatography to obtain FDOL with a purity higher than 99% for use as a comonomer in subsequent reactions. The FDOL obtained in the present invention can be directly used as a polymerization monomer for electrolytes, and FDOL can also be cross-linked with other polymers to form an ion transport network, which is conducive to improving ionic conductivity.

The preparation method for FDOL provided in the present invention realizes synthesis of FDOL in conventional solvents using a foamed transition metal catalyst, enabling reaction to proceed at relatively higher temperature and reducing preparation cost. The preparation method achieves high yield higher than 90%.

The present invention further provides an in-situ solid electrolyte obtained by curing a mixture, wherein
the mixture comprises a fluoro-1,3-dioxolane compound, a free radical polymerization monomer, a first initiator, a second initiator, an electrolyte salt and a non-aqueous organic solvent;
the fluoro-1,3-dioxolane compound is any one selected from Formulas I to X:

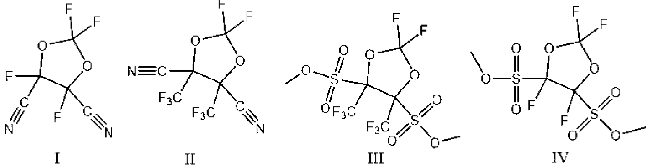

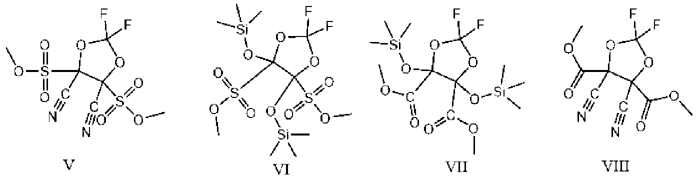

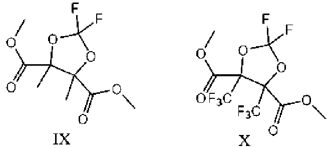

In the present invention, the free radical polymerization monomer is any one or more selected from vinyl ethylene carbonate, methyl vinyl sulfone, ethyl vinyl sulfone, triethylene glycol divinyl ether, methyl methacrylate, vinyl acetate, 1,3-propene sultone, acrylamide, ethyl methacrylate, n-butyl methacrylate, vinylene carbonate, maleic anhydride, succinonitrile, trimethylolpropane triacrylate, ethylene glycol dimethacrylate, vinyl acetate, polyethylene glycol diacrylate and n-butyl acrylate; the first initiator is selected from azo initiators and/or peroxide initiators; the second initiator is any one or more selected from lithium hexafluorophosphate, sodium hexafluorophosphate, lithium tetrafluoroborate, sodium tetrafluoroborate, lithium difluoro(oxalato)borate, sodium difluoro(oxalato)borate, boron trifluoride, niobium pentachloride, titanium tetrachloride, aluminum chloride, ferric chloride and aluminum trifluoromethanesulfonate; the electrolyte salt comprises lithium salt or sodium salt; the lithium salt is any one or more selected from LiPF₆, LiClO₄, LiBF₄, LiPOF₂, LiTFSI, LiFSI, LiODFB and LiBOB; the sodium salt is any one or more selected from NaPF₆, NaClO₄, NaBF₄, NaTFSI, NaFSI and NaODFB; and the non-aqueous organic solvent is any one or more selected from ethylene carbonate, propylene carbonate, diethyl carbonate, ethyl methyl carbonate, dimethyl carbonate and ethylene glycol dimethyl ether.

The present invention further provides a preparation method for the above in-situ solid electrolyte, including the following steps:
S1: mixing a first initiator and a fluoro-1,3-dioxolane compound shown in any one of Formulas I to X to obtain a first polymerization precursor solution; mixing an electrolyte salt, a second initiator, a free radical polymerization monomer and a non-aqueous organic solvent to obtain a second polymerization precursor solution;
S2: mixing the first polymerization precursor solution and the second polymerization precursor solution to obtain a polymerization precursor electrolyte, injecting the polymerization precursor electrolyte into a battery for curing to obtain the in-situ solid electrolyte.

According to the present invention, the first initiator is mixed with a fluoro-1,3-dioxolane compound shown in any one of Formulas I to X to obtain the first polymerization precursor solution. The selection of the first initiator is as described in the above technical solutions, and will not be repeated herein. Meanwhile, the electrolyte salt, the second initiator, the free radical polymerization monomer and the non-aqueous organic solvent are mixed to obtain the second polymerization precursor solution. The specific selection of the electrolyte salt, the second initiator, the free radical polymerization monomer and the non-aqueous organic solvent is as described in the above technical solutions, and will not be repeated herein.

In some embodiments of the present invention, the mass ratio of the non-aqueous organic solvent to the free radical polymerization monomer is 5: (1~10), preferably 5: (2~6).

In the present invention, the first initiator is used to initiate polymerization of the free radical polymerization monomer, and the mass fraction of the first initiator in the first polymerization precursor solution is 0.01%~5%, preferably 0.1%~3%. The second initiator is used to initiate polymerization of the fluoro-1,3-dioxolane compound, and the mass fraction of the second initiator in the second polymerization precursor solution is 0.01%~3%, preferably 0.05%~1%.

In the present invention, the mass fraction of the electrolyte salt in the second polymerization precursor solution is 12%~30%, preferably 15%~25%.

According to the above technical solutions, after preparing the first polymerization precursor solution and the second polymerization precursor solution, the two solutions are mixed to obtain a polymerization precursor electrolyte, which is injected into a battery and cured to obtain the in-situ solid electrolyte.

In some embodiments of the present invention, the mass ratio of the first polymerization precursor solution to the second polymerization precursor solution is 1: (1~5), preferably 1: (2~3).

In the present invention, the above mixing is performed under stirring, wherein the stirring speed is 400 r/min~800 r/min, preferably 500 r/min; the stirring time is 0.5 h~3 h, preferably 1 h~2 h. In the present invention, after the polymerization precursor electrolyte is injected into the battery, the battery is kept standing at room temperature for 12 h~72 h, preferably for 20 h~40 h, so as to complete curing and obtain the in-situ solid electrolyte.

In the preparation method provided by the present invention, the exothermic ionic polymerization of the fluoro-1,3-dioxolane compound at room temperature initiates the polymerization of the free radical polymerization monomer, avoiding non-uniform polymerization caused by external heating. In addition, the free radical monomer polymerization can consume heat released by ion polymerization, resulting in a milder curing process without bubble residues, so that the in-situ solid electrolyte is more uniform in polymerization, and has better contact with an electrode.

To test the performance of the in-situ solid electrolyte prepared by the aforementioned preparation method, the present invention assembled coin cells, with the specific steps as follows.

The positive electrode material, conductive carbon, and polyvinylidene fluoride (PVDF) were mixed at a weight ratio of 96:2:2 and ball-milled for 12 h~24 h. The solvent was N-methylpyrrolidone (NMP), and the solid-liquid ratio was 45%. The resulting slurry was coated onto the surface of aluminum foil and dried at 110°C under forced air for 6 h~12 h, with the areal density of the active material controlled at 5 mg·cm⁻²~15 mg·cm⁻². A 12 mm positive electrode disc was obtained by cutting.

A battery with steel sheet||separator||steel sheet, with blocking electrodes on both sides, was assembled for ionic conductivity testing. A battery with steel sheet||separator||lithium/sodium metal, with lithium metal as the reference electrode and counter electrode and steel sheet as the working electrode, was assembled for voltage window testing. A half cell with positive electrode||separator||lithium/sodium metal negative electrode was assembled for charge-discharge testing.

The polymerization precursor electrolyte was injected into the assembled batteries and cured before corresponding tests. It should be noted that injection amount of the polymerization precursor electrolyte is 80 µL based on active material of positive electrode in coin cells.

Tests showed that the in-situ solid electrolyte exhibited an oxidation voltage higher than 4.5 V and an ionic conductivity higher than 5×10⁻⁴ S/cm. Taking a lithium-ion half-cell as an example, the cycling capacity retention rate after 100 cycles was above 90%.

Given that the in-situ solid electrolyte features high oxidation voltage and ionic conductivity, and the prepared battery exhibits excellent cycling performance, the present invention further provides a solid-state secondary battery comprising a positive electrode, a negative electrode, a separator and an electrolyte, wherein the electrolyte is the in-situ solid electrolyte involved in the aforementioned technical solution. In the present invention, the solid-state secondary battery is a solid-state lithium-ion battery or a solid-state sodium-ion battery.

For solid-state lithium-ion batteries, the positive electrode is prepared by mixing the active material, conductive agent and binder in proportion, adding a solvent to obtain a slurry, coating the slurry on aluminum foil, followed by drying, rolling and cutting. The active material is lithium cobalt oxide (LiCoO₂), lithium iron phosphate (Li₃Fe₂(PO₄)₃), or ternary nickel-cobalt-manganese positive electrode material LiNi_{0.6}Co_{0.2}Mn_{0.2}O₂ (NCM622) or LiNi_{0.8}Co_{0.1}Mn_{0.1}O₂ (NCM811), the conductive agent is conductive carbon black (Super P), and the binder is preferably polyvinylidene fluoride (PVDF). The mass ratio of the active material, conductive agent and binder is 96:2:2. The solvent is N-methylpyrrolidone (NMP). The negative electrode is a lithium metal sheet, and the separator is a polyethylene separator and/or a polypropylene separator.

For solid-state sodium-ion batteries, the positive electrode is prepared by mixing the active material, conductive agent and binder in proportion, adding a solvent to obtain a slurry, coating the slurry on aluminum foil, followed by drying, rolling and cutting. The active material is sodium vanadium phosphate (Na₃V₂ (PO₄)₃), sodium vanadium fluorophosphate (Na₃V₂(PO₄)₂ F₃) or sodium iron sulfate (Na₂Fe (SO₄)₂), the conductive agent is conductive carbon black (Super P), and the binder is polyvinylidene fluoride (PVDF). The mass ratio of the active material, conductive agent and binder is 90:6:4. The solvent is N-methylpyrrolidone (NMP). The negative electrode is a sodium metal sheet, and the separator is a cellulose separator.

In the present invention, the point values listed above are for illustrative purposes only and shall not be construed as limiting, and other values within the numerical ranges are also applicable, which will not be elaborated herein to avoid redundancy.

In the present invention, ambient temperature or room temperature mentioned above refers to 20 °C~30 °C, preferably 25 °C.

The present invention will be further described in detail with the following examples. All experimental raw materials adopted in the following examples of the present invention are general commercially available products.

### Example 1

1. 11.4 g (0.1 mol) of vinyl compound A was dissolved in 39.584 g (0.4 mol) of dichloroethane at -30 °C under an argon atmosphere, and the mixture was then heated to room temperature at a heating rate of 3 °C/min to obtain a homogeneous solution.
2. The above solution containing vinyl compound A was added into a stainless-steel passivation reactor at liquid nitrogen temperature, and the reactor temperature was controlled at - 30 °C. Then 16.4 g (0.2 mol) of CO₂F₂ was condensed into the reactor, and foamed copper was added as a catalyst to facilitate an addition reaction with vinyl compound A for 6 h. The structure of vinyl compound A is shown as follows: 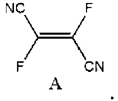
3. Under an argon atmosphere, the reactor was heated at a controlled heating rate to room temperature within 12 h. Dichloroethane was removed by rotary evaporation at 40 °C, and the product was purified by silica gel column chromatography using a mixture of petroleum ether and dichloroethane at a volume ratio of 1:1 as the eluent. Finally, Compound I was isolated with a yield of 92.1% (Yield = Actual Yield / Theoretical Yield). GC-MS (m/z): calcd. for C₅F₄N₂O₂, 196.06, found 195.99. The structure of Compound I is shown as follows: 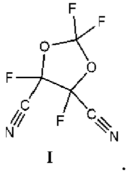
4. Initiator azobisisobutyronitrile was dissolved in Compound I to prepare the first polymerization precursor solution, wherein the mass fraction of azobisisobutyronitrile in the first polymerization precursor solution was 0.01%.
5. Non-aqueous organic solvent diethyl carbonate (DEC) and triethylene glycol divinyl ether were mixed at a mass ratio of 5:2 to prepare a mixed solvent. LiTFSI and initiator LiBF₄ were added into the above mixed solvent and fully dissolved to prepare the second polymerization precursor solution, wherein the mass fractions of LiTFSI and initiator LiBF₄ in the second polymerization precursor solution were 30% and 3%, respectively.
6. The second polymerization precursor solution was added gradually to the first polymerization precursor solution, with a mass ratio of the first polymerization precursor solution to the second polymerization precursor solution of 1:1, stirred at a rotation speed of 500 r·min⁻¹ during mixing and stirred continuously for 0.5 h after mixing to prepare a polymerization precursor electrolyte.
7. NCM811, conductive carbon and PVDF were mixed at a weight ratio of 96:2:2 and ball-milled for 24 h, with NMP as the solvent and a solid-liquid ratio of 45%. The obtained slurry was coated on the surface of aluminum foil and dried under forced air at 110 °C for 6 h, with the areal density of the active material controlled at 5 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 24 h to complete curing. The polymerization reaction formula of Compound I is shown as follows:

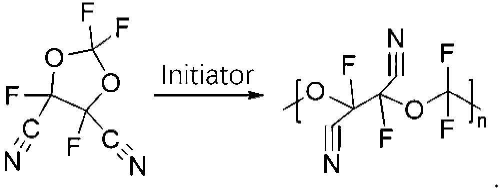

The polymerization reactions of Compounds II~X are similar, except for the branched chain structures.

The in-situ solid electrolyte obtained by curing in this example contains abundant F element, which greatly improves the voltage window of the polymerized electrolyte. Meanwhile, the grafted cyano functional group has a good complexing effect on transition metal ions such as cobalt ions, which can effectively inhibit the damage to the positive electrode interface film caused by the dissolution of transition metal ions in the battery, thereby improving the structural stability of the positive electrode and enhancing the cycling stability of the battery.

### Example 2

1. 38.4 g (0.3 mol) of vinyl compound B was dissolved in 52.8 g (0.6 mol) of CF₂CCl₂ at -35 °C under an argon atmosphere, and the mixture was then heated to room temperature at a heating rate of 2.5 °C·min⁻¹ to obtain a homogeneous solution.
2. The above solution containing vinyl compound B was added into a stainless-steel passivation reactor at liquid nitrogen temperature, and the reactor temperature was controlled at - -30 °C. Then 24.6 g (0.3 mol) of CO₂F₂ was condensed into the reactor, and foamed iron was added as a catalyst to facilitate an addition reaction with vinyl compound B for 14 h. The structure of vinyl compound B is shown as follows: 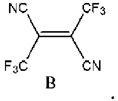
3. Under an argon atmosphere, the reactor was heated at a controlled heating rate to room temperature within 18 hours. CF₂CCl₂ was removed by rotary evaporation at 40 °C, and the product was purified by silica gel column chromatography using a mixture of petroleum ether and dichloroethane at a volume ratio of 1:1 as the eluent. Finally, Compound II was isolated with a yield of 93.3%. GC-MS (m/z): calcd. for C₇F₈N₂O₂, 296.08, found 295.98. The structure of Compound II is shown as follows: 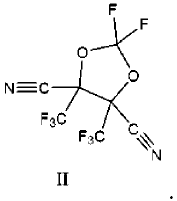
4. Initiator methyl ethyl ketone peroxide was dissolved in Compound II to prepare the first polymerization precursor solution, wherein the mass fraction of the initiator in the first polymerization precursor solution was 0.2%.
5. Non-aqueous organic solvent propylene carbonate (PC) and vinyl acetate monomer were mixed at a mass ratio of 5:8 to prepare a mixed solvent. LiFSI and initiator LiPF₆ were added into the above mixed solvent and fully dissolved to prepare the second polymerization precursor solution, wherein the mass fractions of LiFSI and initiator LiPF₆ in the second polymerization precursor solution were 16% and 0.5%, respectively.
6. The second polymerization precursor solution was added gradually to the first polymerization precursor solution, with a mass ratio of the first polymerization precursor solution to the second polymerization precursor solution of 1:3, stirred at a rotation speed of 800 r·min⁻¹ during mixing and stirred continuously for 3 h after mixing to prepare a polymerization precursor electrolyte.
7. NCM811, conductive carbon and PVDF were mixed at a weight ratio of 96:2:2 and ball-milled for 20 h, with NMP as the solvent and a solid-liquid ratio of 45%. The obtained slurry was coated on the surface of aluminum foil and dried under forced air at 110 °C for 6 h, with the areal density of the active material controlled at 8 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 24 h to complete curing.

The in-situ solid electrolyte obtained by curing in this example contains abundant F element, which can effectively improve the oxidation stability of the electrolyte. Meanwhile, the presence of massive F element can also effectively enhance the flame-retardant effect of the electrolyte, thereby improving the safety performance of the battery. The complexation effect of the cyano functional group on transition metal ions can improve the stability of the positive electrode CEI layer and protect the stable structure of the positive electrode. In addition, the trifluoromethyl group exhibits favorable ionic conductivity and can provide fluoride components for the stability of the negative electrode SEI layer, optimizing the stability of the SEI layer.

### Example 3

1. 140.78 g (0.4 mol) of vinyl compound C was dissolved in 70.4 g (0.8 mol) of CF₂CCl₂ at -35 °C under an argon atmosphere, and the mixture was then heated to room temperature at a heating rate of 5 °C·min⁻¹ to obtain a homogeneous solution.
2. The above solution containing vinyl compound C was added into a stainless-steel passivation reactor at liquid nitrogen temperature, and the reactor temperature was controlled at - 30 °C. Then 98.4 g (1.2 mol) of CO₂F₂ was condensed into the reactor, and foamed iron was added as a catalyst to facilitate an addition reaction with vinyl compound C for 20 h. The structure of vinyl compound C is shown as follows: 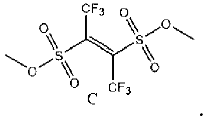
3. Under an argon atmosphere, the reactor was heated at a controlled heating rate to room temperature within 10 hours to vaporize and discharge CF₂CCl₂. The product was purified by silica gel column chromatography using a mixture of petroleum ether and dichloroethane at a volume ratio of 1:1 as the eluent. Finally, Compound III was isolated with a yield of 91.8%. GC-MS (m/z): calcd. for C₇H₆F₈O₈S₂, 434.22, found 433.94. The structure of Compound III is shown as follows: 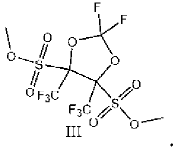
4. Initiator methyl ethyl ketone peroxide was dissolved in Compound III to prepare the first polymerization precursor solution, wherein the mass fraction of the initiator in the first polymerization precursor solution was 2%.
5. Non-aqueous organic solvent propylene carbonate (PC) and vinyl acetate monomer were mixed at a mass ratio of 5:6 to prepare a mixed solvent. LiFSI and initiator LiPF₆ were added into the above mixed solvent and fully dissolved to prepare the second polymerization precursor solution, wherein the mass fractions of LiFSI and initiator LiPF₆ in the second polymerization precursor solution were 12% and 0.01%, respectively.
6. The second polymerization precursor solution was added gradually to the first polymerization precursor solution, with a mass ratio of the first polymerization precursor solution to the second polymerization precursor solution of 1:5, stirred at a rotation speed of 600 r·min⁻¹ during mixing and stirred continuously for 2 h after mixing to prepare a polymerization precursor electrolyte.
7. LiFePO₄, conductive carbon and PVDF were mixed at a weight ratio of 96:2:2 and ball-milled for 18 h, with NMP as the solvent and a solid-liquid ratio of 45%. The obtained slurry was coated on the surface of aluminum foil and dried under forced air at 110 °C for 10 h, with the areal density of the active material controlled at 10 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 20 h to complete curing.

The in-situ solid electrolyte obtained by curing in this example adopts fluoro-1,3-dioxolane compound containing sulfonic acid groups and abundant F element, which can effectively improve the oxidation stability of the electrolyte. Meanwhile, the presence of massive F element can also effectively enhance the flame-retardant effect of the electrolyte, thereby improving the safety performance of the battery. The trifluoromethyl group exhibits favorable ionic conductivity and can provide stable fluoride components for the negative electrode SEI layer, optimizing the stability of the SEI layer. Meanwhile, the functional sulfonic acid group grafted in the compound has a good complexation effect on lithium ions, which can effectively improve the ionic conductivity of the electrolyte.

### Example 4

1. 108.4 g (0.4 mol) of vinyl compound D was dissolved in 27.2 g (4.0 mol) of methanol at -40 °C under an argon atmosphere, and the mixture was then heated to room temperature at a heating rate of 0.1 °C·min⁻¹ to obtain a homogeneous solution.
2. The above solution containing vinyl compound D was added into a stainless-steel passivation reactor at liquid nitrogen temperature, and the reactor temperature was controlled at - 50 °C. Then 49.2 g (0.6 mol) of CO₂F₂ was condensed into the reactor, and foamed nickel was added as a catalyst to facilitate an addition reaction with vinyl compound D for 16 h. The structure of vinyl compound D is shown as follows: 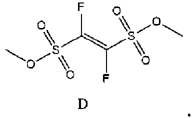
3. Under an argon atmosphere, the reactor was heated at a controlled heating rate to room temperature within 6 h, and methanol was removed by rotary evaporation at 40 °C. The product was purified by silica gel column chromatography using a mixture of petroleum ether and dichloromethane at a volume ratio of 1:1 as the eluent. Finally, Compound IV was isolated with a yield of 94.3%. GC-MS (m/z): calcd. for C₅H₆F₄O₈S₂, 334.21, found 333.94. The structure of Compound IV is shown as follows: 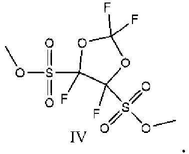
4. Initiator lauroyl peroxide was dissolved in Compound IV to prepare the first polymerization precursor solution, wherein the mass fraction of the initiator in the first polymerization precursor solution was 1%.
5. Non-aqueous organic solvent ethylene carbonate (EC) and ethyl vinyl sulfone monomer were mixed at a mass ratio of 5:4 to prepare a mixed solvent. LiTFSI and initiator LiPF₆ were added into the above mixed solvent and fully dissolved to prepare the second polymerization precursor solution, wherein the mass fractions of LiTFSI and initiator LiPF₆ in the second polymerization precursor solution were 15% and 2%, respectively.
6. The second polymerization precursor solution was added gradually to the first polymerization precursor solution, with a mass ratio of the first polymerization precursor solution to the second polymerization precursor solution of 1:4, stirred at a rotation speed of 550 r·min⁻¹ during mixing and stirred continuously for 2.5 h after mixing to prepare a polymerization precursor electrolyte.
7. LiFePO₄, conductive carbon and PVDF were mixed at a weight ratio of 96:2:2 and ball-milled for 14 h, with NMP as the solvent and a solid-liquid ratio of 45%. The obtained slurry was coated on the surface of aluminum foil and dried under forced air at 110 °C for 8 h, with the areal density of the active material controlled at 12 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 16 h to complete curing.

The in-situ solid electrolyte obtained by curing in this example adopts fluoro-1,3-dioxolane compound containing sulfonic acid groups and abundant F element, which can effectively improve the oxidation stability of the electrolyte. Meanwhile, the presence of massive F element can also effectively enhance the flame-retardant effect of the electrolyte, thereby improving the safety performance of the battery. Meanwhile, the presence of sulfonic acid groups can effectively improve the ionic conductivity of the electrolyte.

### Example 5

1. 133.0 g (0.5 mol) of vinyl compound E was dissolved in 32.0 g (1 mol) of methanol at -40 °C under an argon atmosphere, and the mixture was then heated to room temperature at a heating rate of 0.5 °C·min⁻¹ to obtain a homogeneous solution.
2. The above solution containing vinyl compound E was added into a stainless-steel passivation reactor at liquid nitrogen temperature, and the reactor temperature was controlled at - 45 °C. Then 164.0 g (2 mol) of CO₂F₂ was condensed into the reactor, and foamed nickel was added as a catalyst to facilitate an addition reaction with vinyl compound E for 27 h. The structure of vinyl compound E is shown as follows: 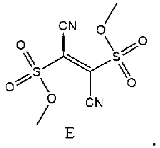
3. Under an argon atmosphere, the reactor was heated at a controlled heating rate to room temperature within 8 h, and methanol was removed by rotary evaporation at 40 °C. The product was purified by silica gel column chromatography using a mixture of petroleum ether and dichloromethane at a volume ratio of 1:1 as the eluent. Finally, Compound V was isolated with a yield of 92.1%. GC-MS (m/z): calcd. for C₇H₆F₂N₂O₈S₂, 348.25, found 347.95. The structure of Compound V is shown as follows: 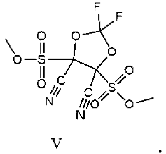
4. Initiator lauroyl peroxide was dissolved in Compound V to prepare the first polymerization precursor solution, wherein the mass fraction of the initiator in the first polymerization precursor solution was 5%.
5. Non-aqueous organic solvent ethylene carbonate (EC) and ethyl vinyl sulfone monomer were mixed at a mass ratio of 5:10 to prepare a mixed solvent. LiTFSI and initiator FeCl₃ were added into the above mixed solvent and fully dissolved to prepare the second polymerization precursor solution, wherein the mass fractions of LiTFSI and initiator FeCl₃ in the second polymerization precursor solution were 30% and 2%, respectively.
6. The second polymerization precursor solution was added gradually to the first polymerization precursor solution, with a mass ratio of the first polymerization precursor solution to the second polymerization precursor solution of 1:1, stirred at a rotation speed of 550 r·min⁻¹ during mixing and stirred continuously for 1.5 h after mixing to prepare a polymerization precursor electrolyte.
7. LCO, conductive carbon and PVDF were mixed at a weight ratio of 96:2:2 and ball-milled for 12 h, with NMP as the solvent and a solid-liquid ratio of 45%. The obtained slurry was coated on the surface of aluminum foil and dried under forced air at 110 °C for 7 h, with the areal density of the active material controlled at 15 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 42 h to complete curing.

The in-situ solid electrolyte obtained by curing in this example adopts fluoro-1,3-dioxolane compound containing sulfonic acid groups and cyano groups. The presence of F element renders the electrolyte high oxidative stability and a certain flame-retardant effect. Meanwhile, the cyano functional groups can effectively complex transition metal ions to protect the stable structure of the positive electrode, and sulfonic acid groups can effectively improve the ionic conductivity of the electrolyte.

### Example 6

1. 162.5 g (0.7 mol) of vinyl compound F was dissolved in 467.25 g (3.5 mol) of chloroform at -45 °C under an argon atmosphere, and the mixture was then heated to room temperature at a heating rate of 1.0 °C·min⁻¹ to obtain a homogeneous solution.
2. The above solution containing vinyl compound F was added into a stainless-steel passivation reactor at liquid nitrogen temperature, and the reactor temperature was controlled at - -30 °C. Then 57.4 g (0.7 mol) of CO₂F₂ was condensed into the reactor, and foamed copper was added as a catalyst to facilitate an addition reaction with vinyl compound F for 48 h. The structure of vinyl compound F is shown as follows: 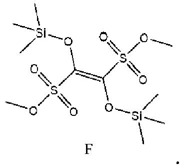
3. Under an argon atmosphere, the reactor was heated at a controlled heating rate to room temperature within 16 h, and chloroform was removed by rotary evaporation at 50 °C. The product was purified by silica gel column chromatography using a mixture of petroleum ether and dichloromethane at a volume ratio of 1:1 as the eluent. Finally, Compound VI was isolated with a yield of 93.1%. GC-MS (m/z): calcd. for C₁₁H₂₄F₂O₁₀S₂Si₂, 474.59, found 474.03. The structure of Compound VI is shown as follows: 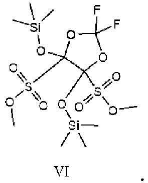
4. Initiator azobisisobutyronitrile was dissolved in Compound VI to prepare the first polymerization precursor solution, wherein the mass fraction of the initiator in the first polymerization precursor solution was 1.2%.
5. Non-aqueous organic solvent ethyl methyl carbonate (EMC) and methyl vinyl sulfone monomer were mixed at a mass ratio of 5:7 to prepare a mixed solvent. LiTFSI and initiator LiODFB were dissolved in the above mixed solvent to prepare the second polymerization precursor solution, wherein the mass fractions of LiTFSI and initiator LiODFB in the second polymerization precursor solution were 25% and 2% respectively.
6. The second polymerization precursor solution was added gradually to the first polymerization precursor solution, with a mass ratio of the first polymerization precursor solution to the second polymerization precursor solution of 1:1.5, stirred at a rotation speed of 800 r·min⁻¹ during mixing and stirred continuously for 2.0 h after mixing to prepare a polymerization precursor electrolyte.
7. NCM622, conductive carbon and PVDF were mixed at a weight ratio of 96:2:2 and ball-milled for 24 h, with NMP as the solvent and a solid-liquid ratio of 45%. The obtained slurry was coated on the surface of aluminum foil and dried under forced air at 110 °C for 6 h, with the areal density of the active material controlled at 10 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 40 h to complete curing.

The in-situ solid electrolyte obtained by curing in this example adopts fluoro-1,3-dioxolane compound containing trimethylsiloxane functional groups. F element can improve the oxidation stability of the polymerized electrolyte, meanwhile, the trimethylsiloxane groups in the solid electrolyte can scavenge excessive HF in the polymerization precursor electrolyte and suppress excessive gas evolution of the battery during electrochemical processes. Furthermore, trimethylsiloxane exhibits excellent stability and exerts a certain flame-retardant effect on the battery. Sulfonic acid groups possess favorable complexation capability toward lithium ion, endowing the electrolyte with superior ionic conductivity.

### Example 7

1. 50.82 g (0.2 mol) of vinyl compound G was dissolved in 119.4 g (1 mol) of chloroform at -45 °C under an argon atmosphere, and the mixture was then heated to room temperature at a heating rate of 5 °C·min⁻¹ to obtain a homogeneous solution.
2. The above solution containing vinyl compound G was added into a stainless-steel passivation reactor at liquid nitrogen temperature, and the reactor temperature was controlled at - 30 °C. Then 82.0 g (1 mol) of CO₂F₂ was condensed into the reactor, and foamed copper was added as a catalyst to facilitate an addition reaction with vinyl compound G for 12 h. The structure of vinyl compound G is shown as follows: 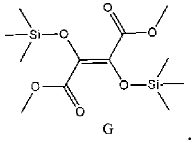
3. Under an argon atmosphere, the reactor was heated at a controlled heating rate to room temperature within 27 h, and chloroform was removed by rotary evaporation at 35 °C. The product was purified by silica gel column chromatography using a mixture of petroleum ether and dichloromethane at a volume ratio of 1:1 as the eluent. Finally, Compound VII was isolated with a yield of 92.1%. GC-MS (m/z): calcd. for C₁₃H₂₄F₂O₈Si₂, 402.49, found 402.10. The structure of Compound VII is shown as follows: 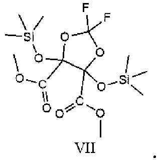
4. Initiator azobisisobutyronitrile was dissolved in Compound VII to prepare the first polymerization precursor solution, wherein the mass fraction of the initiator in the first polymerization precursor solution was 0.1%.
5. Non-aqueous organic solvent ethyl methyl carbonate (EMC) and n-butyl methacrylate monomer were mixed at a mass ratio of 5:3 to prepare a mixed solvent. LiTFSI and initiator LiODFB were dissolved in the above mixed solvent to prepare the second polymerization precursor solution, wherein the mass fractions of LiTFSI and initiator LiODFB in the second polymerization precursor solution were 20% and 0.6% respectively.
6. The second polymerization precursor solution was added gradually to the first polymerization precursor solution, with a mass ratio of the first polymerization precursor solution to the second polymerization precursor solution of 1:2, stirred at a rotation speed of 400 r·min⁻¹ during mixing and stirred continuously for 0.5 h after mixing to prepare a polymerization precursor electrolyte.
7. Lithium iron phosphate (LiFePO₄), conductive carbon and PVDF were mixed at a weight ratio of 96:2:2 and ball-milled for 20 h, with NMP as the solvent and a solid-liquid ratio of 45%. The obtained slurry was coated on the surface of aluminum foil and dried under forced air at 110 °C for 8 h, with the areal density of the active material controlled at 10 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 60 h to complete curing.

The in-situ solid electrolyte obtained via curing in this example adopts fluoro-1,3-dioxolane compound containing trimethylsiloxane and carboxyl functional groups. Not only can it effectively enhance the oxidation stability of the polymerized electrolyte, but also its abundant trimethylsiloxane functional groups can scavenge excessive HF in the polymerization precursor electrolyte and suppress excessive gas generation of the battery during electrochemical processes. Meanwhile, its outstanding structural stability improves the stability of the electrolyte and imparts a certain flame-retardant effect. In addition, the carboxyl groups contained in the solid electrolyte exhibit favorable complexation capability toward lithium ions, which can effectively improve the ionic conductivity of the electrolyte.

### Example 8

1. 58.2 g (0.3 mol) of vinyl compound H was dissolved in 34.8 g (0.6 mol) of acetone at - 25 °C under an argon atmosphere, and the mixture was then heated to room temperature at a heating rate of 2 °C·min⁻¹ to obtain a homogeneous solution.
2. The above solution containing vinyl compound H was added into a stainless-steel passivation reactor at liquid nitrogen temperature, and the reactor temperature was controlled at - 25 °C. Then 49.2 g (0.6 mol) of CO₂F₂ was condensed into the reactor, and foamed iron was added as a catalyst to facilitate an addition reaction with vinyl compound H for 15 h. The structure of vinyl compound H is shown as follows: 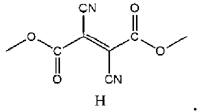
3. Under an argon atmosphere, the reactor was heated at a controlled heating rate to room temperature within 12 h, and acetone was removed by rotary evaporation at 45 °C. The product was purified by silica gel column chromatography using a mixture of petroleum ether and dichloromethane at a volume ratio of 1:1 as the eluent. Finally, Compound VIII was isolated with a yield of 94.1%. GC-MS (m/z): calcd. for C₉H₆F₂N₂O₆, 276.15, found 276.02. The structure of Compound VIII is shown as follows: 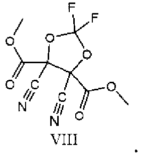
4. Initiator azobisisobutyronitrile was dissolved in Compound VIII to prepare the first polymerization precursor solution, wherein the mass fraction of the initiator in the first polymerization precursor solution was 0.2%.
5. Non-aqueous organic solvent ethylene glycol dimethyl ether (DME) and triethylene glycol divinyl ether monomer were mixed at a mass ratio of 5:1 to prepare a mixed solvent. LiTFSI and initiator LiPF₆ were added into the above mixed solvent and fully dissolved to prepare the second polymerization precursor solution, wherein the mass fractions of LiTFSI and initiator LiPF₆ in the second polymerization precursor solution were 26% and 2% respectively.
6. The second polymerization precursor solution was added gradually to the first polymerization precursor solution, with a mass ratio of the first polymerization precursor solution to the second polymerization precursor solution of 1:3, stirred at a rotation speed of 800 r·min⁻¹ during mixing and stirred continuously for 1.5 h after mixing to prepare a polymerization precursor electrolyte.
7. NCM811, conductive carbon and PVDF were mixed at a weight ratio of 96:2:2 and ball-milled for 24 h, with NMP as the solvent and a solid-liquid ratio of 45%. The obtained slurry was coated on the surface of aluminum foil and dried under forced air at 110 °C for 12 h, with the areal density of the active material controlled at 12 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 72 h to complete curing.

The in-situ solid electrolyte obtained via curing in this example adopts fluoro-1,3-dioxolane compound containing carboxylate and cyano functional groups, which possesses high oxidation stability. Meanwhile, abundant carboxylate functional groups exhibit favorable complexation capability toward lithium ions, effectively improving the ionic conductivity of the electrolyte. Furthermore, cyano functional groups show strong complexation capability toward transition metal ions, which can effectively inhibit the damage to positive electrode stability caused by the dissolution of transition metal ions.

### Example 9

1. 68.8 g (0.4 mol) of vinyl compound I was dissolved in 129.8 g (1.8 mol) of methyl ethyl ketone at -40 °C under an argon atmosphere, and the mixture was then heated to room temperature at a heating rate of 5 °C·min⁻¹ to obtain a homogeneous solution.
2. The above solution containing vinyl compound I was added into a stainless-steel passivation reactor at liquid nitrogen temperature, and the reactor temperature was controlled at - 40 °C. Then 82.0 g (1 mol) of CO₂F₂ was condensed into the reactor, and foamed metal was added as a catalyst to facilitate an addition reaction with vinyl compound I for 18 h. The structure of vinyl compound I is shown as follows: 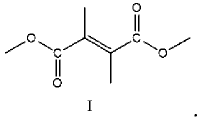
3. Under an argon atmosphere, the reactor was heated at a controlled heating rate to room temperature within 48 h, and methyl ethyl ketone was removed by rotary evaporation at 40 °C. The product was purified by silica gel column chromatography using a mixture of petroleum ether and dichloromethane at a volume ratio of 1:1 as the eluent. Finally, Compound IX was isolated with a yield of 95.0%. GC-MS (m/z): calcd. for C₉H₁₂F₂O₆, 254.19, found 254.06. The structure of Compound IX is shown as follows: 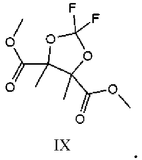
4. Initiator azobisisobutyronitrile was dissolved in Compound IX to prepare the first polymerization precursor solution, wherein the mass fraction of the initiator in the first polymerization precursor solution was 1%.
5. Non-aqueous organic solvent diethyl carbonate (DEC) and trimethylolpropane triacrylate monomer were mixed at a mass ratio of 5:2 to prepare a mixed solvent. LiFSI and initiator LiBF₄ were added into the above mixed solvent to prepare the second polymerization precursor solution, wherein the mass fractions of LiFSI and initiator LiBF₄ in the second polymerization precursor solution were 22% and 0.05% respectively.
6. The second polymerization precursor solution was added gradually to the first polymerization precursor solution, with a mass ratio of the first polymerization precursor solution to the second polymerization precursor solution of 1:3.5, stirred at a rotation speed of 800 r·min⁻¹ during mixing and stirred continuously for 3 h after mixing to prepare a polymerization precursor electrolyte.
7. LiFePO₄, conductive carbon and PVDF were mixed at a weight ratio of 90:6:4 and ball-milled for 72 h, with NMP as the solvent and a solid-liquid ratio of 45%. The obtained slurry was coated on the surface of aluminum foil and dried under forced air at 110 °C for 24 h, with the areal density of the active material controlled at 10 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 12 h to complete curing.

The in-situ solid electrolyte obtained via curing in this example adopts fluoro-1,3-dioxolane compound containing carboxylate groups. The present of F element renders the electrolyte high oxidation stability and certain flame-retardant effect. Meanwhile, abundant carboxylate groups possess favorable complexation capability toward lithium/sodium ions, which can effectively enhance the ionic conductivity of the electrolyte.

### Example 10

1. 140.0 g (0.5 mol) of vinyl compound J was dissolved in 144.2 g (2.0 mol) of methyl ethyl ketone at -50 °C under an argon atmosphere, and the mixture was then heated to room temperature at a heating rate of 0.1 °C·min⁻¹ to obtain a homogeneous solution.
2. The above solution containing vinyl compound J was added into a stainless-steel passivation reactor at liquid nitrogen temperature, and the reactor temperature was controlled at - 40 °C. Then 123.0 g (1.5 mol) of CO₂F₂ was condensed into the reactor, and foamed metal was added as a catalyst to facilitate an addition reaction with vinyl compound J for 24 h. The structure of vinyl compound J is shown as follows: 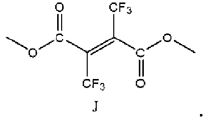
3. Under an argon atmosphere, the reactor was heated at a controlled heating rate to room temperature within 12 h, and methyl ethyl ketone was removed by rotary evaporation at 40 °C. The product was purified by silica gel column chromatography using a mixture of petroleum ether and dichloromethane at a volume ratio of 1:1 as the eluent. Finally, Compound X was isolated with a yield of 95.1%. GC-MS (m/z): calcd. for C₉H₆F₈O₆, 362.13, found 362.00. The structure of Compound X is shown as follows: 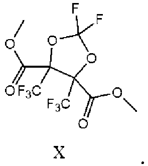
4. Initiator azobisisobutyronitrile was dissolved in Compound X to prepare the first polymerization precursor solution, wherein the mass fraction of the initiator in the first polymerization precursor solution was 0.04%.
5. Non-aqueous organic solvent ethylene carbonate (EC) and ethylene glycol dimethacrylate monomer were mixed at a mass ratio of 5:1 to prepare a mixed solvent. LiFSI and initiator LiBF₄ were dissolved in the above mixed solvent to prepare the second polymerization precursor solution, wherein the mass fractions of LiFSI and initiator LiBF₄ in the second polymerization precursor solution were 16% and 0.05% respectively.
6. The second polymerization precursor solution was added gradually to the first polymerization precursor solution, with a mass ratio of the first polymerization precursor solution to the second polymerization precursor solution of 1:3, stirred at a rotation speed of 400 r·min⁻¹ during mixing and stirred continuously for 1.5 h after mixing to prepare a polymerization precursor electrolyte.
7. LiFePO₄, conductive carbon and PVDF were mixed at a weight ratio of 96:2:2 and ball-milled for 12 h, with NMP as the solvent and a solid-liquid ratio of 45%. The obtained slurry was coated on the surface of aluminum foil and dried under forced air at 110 °C for 6 h, with the areal density of the active material controlled at 8 mg·cm⁻². A 12 mm positive electrode disc was obtained by cutting.
8. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
9. The polymerization precursor electrolyte was injected into each battery and allowed to stand at room temperature for 20 h to complete curing.
The in-situ solid electrolyte obtained via curing in this example adopts fluoro-1,3-dioxolane compound containing carboxylate and trifluoromethyl functional groups. Abundant F element can effectively improve the oxidation stability of the electrolyte. Meanwhile, the presence of massive F element can also significantly enhance the flame-retardant effect of the electrolyte, thereby boosting the safety performance of the battery. The grafted trifluoromethyl groups exhibit favorable ionic conductivity, and can provide fluoride components for the stability of the negative SEI layer, optimizing the stability of the SEI layer.

### Example 11

1. Compound I and Compound IV were mixed at a mass ratio of 1:1 to prepare mixed solvent 1. Initiator azobisisobutyronitrile was dissolved in mixed solvent 1 to prepare the first polymerization precursor solution, wherein the mass fraction of the initiator in the first polymerization precursor solution was 0.04%.
2. Non-aqueous organic solvent ethylene carbonate (EC) and ethylene glycol dimethacrylate monomer were mixed at a mass ratio of 5:1 to prepare a mixed solvent. LiTFSI and initiator LiBF₄ were dissolved in the above mixed solvent to prepare the second polymerization precursor solution, wherein the mass fractions of LiTFSI and initiator LiBF₄ in the second polymerization precursor solution were 16% and 0.1% respectively.
3. The second polymerization precursor solution was added gradually to the first polymerization precursor solution, with a mass ratio of the first polymerization precursor solution to the second polymerization precursor solution of 1:4, stirred at a rotation speed of 700 r·min⁻¹ during mixing and stirred continuously for 1.5 h after mixing to prepare a polymerization precursor electrolyte.
4. Electrode preparation and coin cell assembly were the same as those in Example 10. The polymerization precursor electrolyte was injected into each battery and allowed to stand at room temperature for 20 h to complete curing. The in-situ solid electrolyte obtained via curing in this example adopts fluoro-1,3-dioxolane compounds containing cyano and sulfonic acid functional groups. Abundant F element can effectively improve the oxidation stability of the electrolyte. Meanwhile, the presence of massive F element can also significantly enhance the flame-retardant effect of the electrolyte, thereby boosting the safety performance of the battery. Meanwhile, the abundant cyano groups exhibit favorable complexation effect on transition metal ions such as cobalt ions, which can effectively inhibit the damage to the positive electrode interface film caused by the dissolution of transition metal ions during excessive gas generation of the battery in electrochemical processes, thus improving the structural stability of the positive electrode and enhancing the cycling stability of the battery. Sulfonic acid functional groups possess favorable complexation capability toward lithium/sodium ions, which can effectively enhance the ionic conductivity of the electrolyte.

### Example 12

1. Initiator azobisisobutyronitrile was dissolved in Compound I to prepare the first polymerization precursor solution, wherein the mass fraction of azobisisobutyronitrile in the first polymerization precursor solution was 0.01%.
2. Non-aqueous organic solvent diethyl carbonate (DEC) and triethylene glycol divinyl ether were mixed at a mass ratio of 5:2 to prepare a mixed solvent. NaClO₄ and initiator NaBF₄ were added into the above mixed solvent and fully dissolved to prepare the second polymerization precursor solution, wherein the mass fractions of NaClO₄ and initiator NaBF₄ in the second polymerization precursor solution were 30% and 3% respectively.
3. The second polymerization precursor solution was added gradually to the first polymerization precursor solution, with a mass ratio of the first polymerization precursor solution to the second polymerization precursor solution of 1:1, stirred at a rotation speed of 500 r·min⁻¹ during mixing and stirred continuously for 0.5 h after mixing to prepare a polymerization precursor electrolyte.
4. Sodium vanadium phosphate (Na₃V₂(PO₄)₃), conductive carbon and PVDF were mixed at a weight ratio of 90:6:4 and ball-milled for 20 h, with NMP as the solvent and a solid-liquid ratio of 45%. The obtained slurry was coated on the surface of aluminum foil and dried under forced air at 110 °C for 8 h, with the areal density of the active material controlled at 10 mg·cm⁻². A 12 mm positive electrode disc was obtained by cutting.
5. A battery with steel sheet||separator||steel sheet, with blocking electrodes on both sides, was assembled for ionic conductivity testing. A battery with steel sheet||separator||sodium metal, with sodium metal as the reference electrode and counter electrode and steel sheet as the working electrode, was assembled for voltage window testing. A half cell with positive electrode||separator||sodium metal negative electrode was assembled for charge-discharge testing.
6. The polymerization precursor electrolyte was injected into each battery and allowed to stand at room temperature for 16 h to complete curing.

The in-situ solid electrolyte obtained via curing in this example contains abundant F element, which greatly improves the voltage window of the polymerized electrolyte. Meanwhile, the grafted cyano functional group exhibit favorable complexation effect on transition metal ions such as cobalt ions, which can effectively inhibit the damage to the positive electrode interface film caused by the dissolution of transition metal ions in the battery, thereby improving the structural stability of the positive electrode and enhancing the cycling stability of the battery.

### Example 13

1. Initiator azobisisobutyronitrile was dissolved in Compound II to prepare the first polymerization precursor solution, wherein the mass fraction of the initiator in the first polymerization precursor solution was 0.1%.
2. Non-aqueous organic solvent ethyl methyl carbonate (EMC) and n-butyl methacrylate monomer were mixed at a mass ratio of 5:3 to prepare a mixed solvent. NaClO₄ and initiator NaODFB were added into the above mixed solvent and fully dissolved to prepare the second polymerization precursor solution, wherein the mass fractions of NaClO₄ and initiator NaODFB in the second polymerization precursor solution were 20% and 0.6% respectively.
3. The second polymerization precursor solution was added gradually to the first polymerization precursor solution, with a mass ratio of the first polymerization precursor solution to the second polymerization precursor solution of 1:2, stirred at a rotation speed of 400 r·min⁻¹ during mixing and stirred continuously for 0.5 h after mixing to prepare a polymerization precursor electrolyte.
4. Sodium vanadium phosphate (Na₃V₂(PO₄)₃) was used as the positive electrode, and electrode preparation and cell assembly were the same as those in Example 12.
5. The polymerization precursor electrolyte was injected into each battery and allowed to stand at room temperature for 20 h to complete curing.

The in-situ solid electrolyte obtained via curing in this example contains abundant F element, which can effectively improve the oxidation stability of the electrolyte. Meanwhile, the presence of massive F element can also significantly enhance the flame-retardant effect of the electrolyte, thereby boosting the safety performance of the battery. The complexation effect of cyano functional groups on transition metal ions can stabilize the positive electrode CEI layer and protect the structural stability of the positive electrode. In addition, trifluoromethyl groups exhibit favorable ionic conductivity and can provide fluoride components for the stability of the negative electrode SEI layer, optimizing the stability of the SEI layer.

### Example 14

1. Initiator azobisisobutyronitrile was dissolved in Compound III to prepare the first polymerization precursor solution, wherein the mass fraction of the initiator in the first polymerization precursor solution was 0.2%.
2. Non-aqueous organic solvent ethylene glycol dimethyl ether (DME) and triethylene glycol divinyl ether monomer were mixed at a mass ratio of 5:1 to prepare a mixed solvent. NaClO₄ and initiator NaBF₄ were added into the above mixed solvent and fully dissolved to prepare the second polymerization precursor solution, wherein the mass fractions of NaClO₄ and initiator NaBF₄ in the second polymerization precursor solution were 26% and 2% respectively.
3. The second polymerization precursor solution was added gradually to the first polymerization precursor solution, with a mass ratio of the first polymerization precursor solution to the second polymerization precursor solution of 1:3, stirred at a rotation speed of 800 r·min⁻¹ during mixing and stirred continuously for 1.5 h after mixing to prepare a polymerization precursor electrolyte.
4. Sodium vanadium phosphate (Na₃V₂(PO₄)₃) was used as the positive electrode, and the electrode preparation procedure was the same as that in Example 12, except that the areal density of the active material was controlled at 12 mg·cm⁻².
5. The coin cell assembly method was identical to that in Example 12.
6. The polymerization precursor electrolyte was injected into each battery and allowed to stand at room temperature for 72 h to complete curing.

The in-situ solid electrolyte obtained via curing in this example adopts fluoro-1,3-dioxolane compound containing sulfonic acid groups. Abundant F element can effectively improve the oxidation stability of the electrolyte. Meanwhile, the presence of massive F element can also significantly enhance the flame-retardant effect of the electrolyte, thereby boosting the safety performance of the battery. Trifluoromethyl groups exhibit favorable ionic conductivity and can provide fluoride components for the stability of the negative electrode SEI layer, optimizing the stability of the SEI layer. Meanwhile, the functional sulfonic acid groups grafted on the compound possess favorable complexation capability toward sodium ions, which can effectively enhance the ionic conductivity of the electrolyte.

### Example 15

1. Initiator azobisisobutyronitrile was dissolved in Compound IV to prepare the first polymerization precursor solution, wherein the mass fraction of the initiator in the first polymerization precursor solution was 1%.
2. Non-aqueous organic solvent diethyl carbonate (DEC) and trimethylolpropane triacrylate monomer were mixed at a mass ratio of 5:2 to prepare a mixed solvent. NaFSI and initiator NaBF₄ were added into the above mixed solvent to prepare the second polymerization precursor solution, wherein the mass fractions of NaFSI and initiator NaBF₄ in the second polymerization precursor solution were 22% and 0.05% respectively.
3. The second polymerization precursor solution was added gradually to the first polymerization precursor solution, with a mass ratio of the first polymerization precursor solution to the second polymerization precursor solution of 1:3.5, stirred at a rotation speed of 800 r·min⁻¹ during mixing and stirred continuously for 3 h after mixing to prepare a polymerization precursor electrolyte.
4. Sodium vanadium fluorophosphate (Na₃V₂(PO₄)₂F₃), conductive carbon and PVDF were mixed at a weight ratio of 90:6:4 and ball-milled for 72 h, with NMP as the solvent and a solid-liquid ratio of 45%. The obtained slurry was coated on the surface of aluminum foil and dried under forced air at 110 °C for 24 h, with the areal density of the active material controlled at 10 mg·cm⁻². A 12 mm positive electrode disc was obtained by cutting.
5. The coin cell assembly method was identical to that in Example 12.
6. The polymerization precursor electrolyte was injected into each battery and allowed to stand at room temperature for 12 h to complete curing.

The in-situ solid electrolyte obtained via curing in this example adopts fluoro-1,3-dioxolane compound containing sulfonic acid groups. Abundant F element can effectively improve the oxidation stability of the electrolyte. Meanwhile, the presence of massive F element can also significantly enhance the flame-retardant effect of the electrolyte, thereby boosting the safety performance of the battery. Meanwhile, the presence of sulfonic acid groups can effectively enhance the ionic conductivity of the electrolyte.

### Example 16

1. Initiator azobisisoheptonitrile was dissolved in Compound V to prepare the first polymerization precursor solution, wherein the mass fraction of azobisisoheptonitrile in the first polymerization precursor solution was 0.04%.
2. Non-aqueous organic solvent propylene carbonate (PC) and ethylene glycol dimethacrylate monomer were mixed at a mass ratio of 5:1 to prepare a mixed solvent. NaFSI and initiator NaBF₄ were dissolved in the above mixed solvent to prepare the second polymerization precursor solution, wherein the mass fractions of NaFSI and initiator NaBF₄ in the second polymerization precursor solution were 16% and 0.05% respectively.
3. The second polymerization precursor solution was added gradually to the first polymerization precursor solution, with a mass ratio of the first polymerization precursor solution to the second polymerization precursor solution of 1:3, stirred at a rotation speed of 400 r·min⁻¹ during mixing and stirred continuously for 1.5 h after mixing to prepare a polymerization precursor electrolyte.
4. Sodium vanadium fluorophosphate (Na₃V₂(PO₄)₂F₃) was used as the positive electrode, and the electrode preparation procedure was the same as that in Example 15, except that the areal density of the active material was controlled at 8 mg·cm⁻².
5. The coin cell assembly method was identical to that in Example 12.
6. The polymerization precursor electrolyte was injected into each battery and allowed to stand at room temperature for 20 h to complete curing.

The in-situ solid electrolyte obtained via curing in this example adopts fluoro-1,3-dioxolane compound containing sulfonic acid groups. Abundant F element can effectively improve the oxidation stability of the electrolyte. Meanwhile, the presence of massive F element can also significantly enhance the flame-retardant effect of the electrolyte, thereby boosting the safety performance of the battery. Meanwhile, the presence of sulfonic acid groups can effectively enhance the ionic conductivity of the electrolyte.

### Example 17

1. Initiator lauroyl peroxide was dissolved in Compound VI to prepare the first polymerization precursor solution, wherein the mass fraction of lauroyl peroxide in the first polymerization precursor solution was 0.2%.
2. Non-aqueous organic solvent propylene carbonate (PC) and vinyl acetate monomer were mixed at a mass ratio of 5:8 to prepare a mixed solvent. NaFSI and initiator NaBF₄ were dissolved in the above mixed solvent to prepare the second polymerization precursor solution, wherein the mass fractions of NaFSI and initiator NaBF₄ in the second polymerization precursor solution were 16% and 0.5% respectively.
3. The second polymerization precursor solution was added gradually to the first polymerization precursor solution, with a mass ratio of the first polymerization precursor solution to the second polymerization precursor solution of 1:3, stirred at a rotation speed of 700 r·min⁻¹ during mixing and stirred continuously for 1.5 h after mixing to prepare a polymerization precursor electrolyte.
4. Sodium iron sulfate (Na₂Fe(SO₄)₂) was used as the positive electrode, and the electrode preparation procedure was the same as that in Example 12, except that the areal density of the active material was controlled at 8 mg·cm⁻².
5. The coin cell assembly method was identical to that in Example 12.
6. The polymerization precursor electrolyte was injected into each battery and allowed to stand at room temperature for 24 h to complete curing.

The in-situ solid electrolyte obtained via curing in this example adopts fluoro-1,3-dioxolane compound containing trimethylsiloxane functional groups. F element can improve the oxidation stability of the polymerized electrolyte, meanwhile, the trimethylsiloxane groups in the solid electrolyte can scavenge excessive HF in the polymerization precursor electrolyte and suppress excessive gas evolution of the battery during electrochemical processes. Furthermore, trimethylsiloxane exhibits excellent stability and exerts a certain flame-retardant effect on the battery. Sulfonic acid groups possess favorable complexation capability toward sodium ions, endowing the electrolyte with superior ionic conductivity.

### Example 18

1. Initiator methyl ethyl ketone peroxide was dissolved in Compound VII to prepare the first polymerization precursor solution, wherein the mass fraction of methyl ethyl ketone peroxide in the first polymerization precursor solution was 2%.
2. Non-aqueous organic solvent propylene carbonate (PC) and vinyl acetate monomer were mixed at a mass ratio of 5:8 to prepare a mixed solvent. NaFSI and initiator NaBF₄ were dissolved in the above mixed solvent to prepare the second polymerization precursor solution, wherein the mass fractions of NaFSI and initiator NaBF₄ in the second polymerization precursor solution were 12% and 0.01% respectively.
3. The second polymerization precursor solution was added gradually to the first polymerization precursor solution, with a mass ratio of the first polymerization precursor solution to the second polymerization precursor solution of 1:5, stirred at a rotation speed of 600 r·min⁻¹ during mixing and stirred continuously for 2 h after mixing to prepare a polymerization precursor electrolyte.
4. Sodium iron sulfate (Na₂Fe (SO₄)₂) was used as the positive electrode, and electrode preparation was the same as that in Example 12.
5. The coin cell assembly method was identical to that in Example 12.
6. The polymerization precursor electrolyte was injected into each battery and allowed to stand at room temperature for 22 h to complete curing.

The in-situ solid electrolyte obtained via curing in this example adopts fluoro-1,3-dioxolane compound containing trimethylsiloxane and carboxyl functional groups. Not only can it effectively enhance the oxidation stability of the polymerized electrolyte, but also its abundant trimethylsiloxane functional groups can scavenge excessive HF in the polymerization precursor electrolyte and suppress excessive gas generation of the battery during electrochemical processes. Meanwhile, its outstanding structural stability improves the stability of the electrolyte and imparts a certain flame-retardant effect. In addition, the carboxyl groups contained in the solid electrolyte exhibit favorable complexation capability toward sodium ions, which can effectively improve the ionic conductivity of the electrolyte.

### Example 19

1. Initiator azobisisobutyronitrile was dissolved in Compound VIII to prepare the first polymerization precursor solution, wherein the mass fraction of the initiator in the first polymerization precursor solution was 2%.
2. Non-aqueous organic solvent propylene carbonate (PC) and vinyl acetate monomer were mixed at a mass ratio of 5:8 to prepare a mixed solvent. NaFSI and initiator NaBF₄ were dissolved in the above mixed solvent to prepare the second polymerization precursor solution, wherein the mass fractions of NaFSI and initiator NaBF₄ in the second polymerization precursor solution were 12% and 0.01% respectively.
3. The second polymerization precursor solution was added gradually to the first polymerization precursor solution, with a mass ratio of the first polymerization precursor solution to the second polymerization precursor solution of 1:5, stirred at a rotation speed of 600 r·min⁻¹ during mixing and stirred continuously for 2 h to prepare a polymerization precursor electrolyte.
4. Sodium iron sulfate (Na₂Fe(SO₄)₂) was used as the positive electrode, and the electrode preparation procedure was the same as that in Example 12.
5. The coin cell assembly method was identical to that in Example 12.
6. The polymerization precursor electrolyte was injected into each battery and allowed to stand at room temperature for 22 h to complete curing.

The in-situ solid electrolyte obtained via curing in this example adopts fluoro-1,3-dioxolane compound containing carboxylate and cyano functional groups, which possesses high oxidation stability. Meanwhile, abundant carboxylate groups exhibit favorable complexation capability toward sodium ions, effectively improving the ionic conductivity of the electrolyte. Furthermore, cyano functional groups show strong complexation capability toward transition metal ions, which can effectively inhibit the damage to positive electrode stability caused by the dissolution of transition metal ions.

### Example 20

1. Initiator methyl ethyl ketone peroxide was dissolved in Compound VI to prepare the first polymerization precursor solution, wherein the mass fraction of methyl ethyl ketone peroxide in the first polymerization precursor solution was 1.2%.
2. Non-aqueous organic solvent propylene carbonate (PC) and methyl vinyl sulfone monomer were mixed at a mass ratio of 5:7 to prepare a mixed solvent. LiTFSI and initiator LiODFB were dissolved in the above mixed solvent to prepare the second polymerization precursor solution, wherein the mass fractions of LiTFSI and initiator LiODFB in the second polymerization precursor solution were 25% and 2% respectively.
3. The second polymerization precursor solution was added gradually to the first polymerization precursor solution, with a mass ratio of the first polymerization precursor solution to the second polymerization precursor solution of 1:1.5, stirred at a rotation speed of 800 r·min⁻¹ during mixing and stirred continuously for 2.0 h to prepare a polymerization precursor electrolyte.
4. Positive electrode material NCM811, conductive agent carbon black (Super P) and binder polyvinylidene fluoride (PVDF, for an NMP solution with a mass fraction of 5%) were weighed and mixed at a mass ratio of 96.5:1.5:2. After mixing, an appropriate amount of NMP was added to adjust the theoretical solid content to 55%, and the mixture was homogenized using a vacuum defoaming machine to obtain positive electrode slurry. The slurry was uniformly coated onto aluminum foil with a thickness of 17 µm, followed by drying, rolling and cutting to obtain positive electrode sheets of 50 mm × 70 mm.
5. Negative electrode material artificial graphite, conductive agent Super P, thickener sodium carboxymethyl cellulose (CMC, for a solution with mass fraction of 1.5% in deionized water) and binder styrene-butadiene rubber (SBR, for a solution with mass fraction of 48% in deionized water) were mixed at a mass ratio of 95:1:1.5:2.5. After mixing, deionized water was added to adjust the theoretical solid content to 52%, and the mixture was homogenized using a vacuum defoaming machine to obtain negative electrode slurry. The slurry was uniformly coated onto copper foil with a thickness of 17 µm, followed by drying, rolling and cutting to obtain negative electrode sheets of 52 mm × 72 mm. The N/P ratio of positive electrode to negative electrode was 1.1.
6. Polyethylene separator was cut into pieces of 55 mm × 75 mm and vacuum baked at 70 °C for 48 h to remove moisture.
7. Pouch stacked cells were fabricated in an environment with dew point ≤ -45 °C. Positive electrode sheets, separators and negative electrode sheets were stacked sequentially with tabs of positive and negative electrodes on the same side, with separators isolating positive and negative electrode sheets to form bare cells. The bare cells were placed in aluminum-plastic packaging films, vacuum baked at 90 °C for 12 h, and cooled to below 40 °C before injection of the prepared polymerization precursor electrolyte. Subsequent processes including encapsulation, impregnation, formation, aging, secondary vacuum encapsulation, and capacity sorting.

### Example 21

1. Initiator azobisisobutyronitrile was dissolved in Compound VII to prepare the first polymerization precursor solution, wherein the mass fraction of the initiator in the first polymerization precursor solution was 0.1%.
2. Non-aqueous organic solvent propylene carbonate (PC) and methyl methacrylate monomer were mixed at a mass ratio of 5:3 to prepare a mixed solvent. LiTFSI and initiator LiBF₄ were dissolved in the above mixed solvent to prepare the second polymerization precursor solution, wherein the mass fractions of LiTFSI and initiator LiBF₄ in the second polymerization precursor solution were 20% and 0.6% respectively.
3. The second polymerization precursor solution was added gradually to the first polymerization precursor solution, with a mass ratio of the first polymerization precursor solution to the second polymerization precursor solution of 1:2, stirred at a rotation speed of 800 r·min⁻¹ during mixing and stirred continuously for 1.0 h to prepare the polymerization precursor electrolyte.
4. The battery fabrication and testing methods were the same as those in Example 20.

### Comparative Example 1

1. As electrolyte solvents, dimethyl carbonate (DMC) and ethylene carbonate (EC), were mixed at a mass ratio of 4:3 to prepare a mixed solvent. LiTFSI and initiator LiPF₆ were dissolved in the above mixed solvent with sufficient dissolution to prepare a base electrolyte containing initiator, wherein the mass fractions of the mixed solvent, LiTFSI and initiator LiPF₆ in the base electrolyte were 69%, 30% and 1%, respectively.
2. The base electrolyte containing initiator in step 1 was added gradually to DOL, with a mass ratio of the base electrolyte to DOL of 1:2. The polymerization precursor solution was stirred at a rotation speed of 600 r·min⁻¹ during mixing, and stirring was continued for 1 h after mixing to prepare a polymerization precursor electrolyte.
3. NCM811, conductive carbon and PVDF were mixed at a weight ratio of 90:6:4 and ball-milled for 24 h, with NMP as the solvent and a solid-liquid ratio of 45%. The obtained slurry was coated on the surface of aluminum foil and dried under forced air at 110 °C for 6 h, with the areal density of the active material controlled at 10 mg·cm⁻². A 12 mm positive electrode disc was obtained by cutting.
4. A battery with steel sheet||separator||steel sheet, with blocking electrodes on both sides, was assembled for ionic conductivity testing. A battery with steel sheet||separator||lithium metal, with lithium metal as the reference electrode and counter electrode and steel sheet as the working electrode, was assembled for voltage window testing. A half cell with positive electrode||separator||lithium metal negative electrode was assembled for charge-discharge testing.
5. The polymerization precursor electrolyte was injected into each battery and allowed to stand at room temperature for 20 h to complete curing.

### Comparative Example 2

1. Initiator azobisisobutyronitrile was dissolved in 1,3-dioxolane (DOL) to prepare the first polymerization precursor solution, wherein the mass fraction of the initiator in the first polymerization precursor solution was 0.2%.
2. Non-aqueous organic solvent ethylene carbonate (EC) and ethylene glycol dimethacrylate monomer were mixed at a mass ratio of 5:2 to prepare a mixed solvent. LiTFSI and initiator LiBF₄ were added into the above mixed solvent to prepare the second polymerization precursor solution, wherein the mass fractions of LiTFSI and initiator LiBF₄ in the second polymerization precursor solution were 23% and 0.05%, respectively.
3. The second polymerization precursor solution was added gradually to the first polymerization precursor solution, with a mass ratio of the first polymerization precursor solution to the second polymerization precursor solution of 1:3.5. The polymerization precursor solution was stirred at a rotation speed of 500 r·min⁻¹ during mixing and stirred continuously for 3 h after mixing to prepare a polymerization precursor electrolyte.
4. The methods for electrode preparation and battery assembly and testing were the same as those in Comparative Example 1.
5. The polymerization precursor electrolyte was injected into each battery and allowed to stand at room temperature for 20 h to complete curing.

### Comparative Example 3

1. As electrolyte solvents, dimethyl carbonate (DMC) and ethylene carbonate (EC) were mixed at a mass ratio of 4:3 to prepare a mixed solvent. LiTFSI and initiator LiPF₆ were dissolved in the above mixed solvent with sufficient dissolution to prepare a base electrolyte containing initiator, wherein the mass fractions of the mixed solvent, LiTFSI and initiator LiPF₆ in the base electrolyte were 69%, 30% and 1%, respectively.
2. The base electrolyte containing initiator in step 1 was added gradually to perfluoro-1,3-dioxolane, with a mass ratio of the base electrolyte to perfluoro-1,3-dioxolane of 1:2. The polymerization precursor solution was stirred at a rotation speed of 1200 r·min⁻¹ during mixing, and stirring was continued for 1 h after mixing to prepare a polymerization precursor electrolyte.
3. The methods for electrode preparation and battery assembly were the same as those in Comparative Example 1.
4. The polymerization precursor electrolyte was injected into each battery and allowed to stand at room temperature for 24 h to complete curing.

### Comparative Example 4

1. Initiator azobisisobutyronitrile was dissolved in perfluoro-1,3-dioxolane to prepare the first polymerization precursor solution, wherein the mass fraction of the initiator in the first polymerization precursor solution was 0.2%.
2. Non-aqueous organic solvent ethylene carbonate (EC) and ethylene glycol dimethacrylate monomer were mixed at a mass ratio of 5:2 to prepare a mixed solvent. LiTFSI and initiator LiBF₄ were added into the above mixed solvent to prepare the second polymerization precursor solution, wherein the mass fractions of LiTFSI and initiator LiBF₄ in the second polymerization precursor solution were 23% and 0.05%, respectively.
3. The second polymerization precursor solution was added gradually to the first polymerization precursor solution, with a mass ratio of the first polymerization precursor solution to the second polymerization precursor solution of 1:3.5. The polymerization precursor solution was stirred at a rotation speed of 500 r·min⁻¹ during mixing and stirred continuously for 3 h after mixing to prepare a polymerization precursor electrolyte.
4. The methods for electrode preparation and battery assembly were the same as those in Comparative Example 1.
5. The polymerization precursor electrolyte was injected into each battery and allowed to stand at room temperature for 20 h to complete curing.

### Comparative Example 5

1. As electrolyte solvents, dimethyl carbonate (DMC) and ethylene carbonate (EC) were mixed at a mass ratio of 4:3 to prepare a mixed solvent. Lithium salt LiTFSI was dissolved in the above mixed solvent with sufficient dissolution to prepare Comparative Electrolyte 5, wherein the mass fractions of the mixed solvent and LiTFSI in the electrolyte were 87% and 13%, respectively.
2. NCM811, conductive carbon and PVDF were mixed at a weight ratio of 90:6:4 and ball-milled for 24 h, with NMP as the solvent and a solid-liquid ratio of 45%. The obtained slurry was coated on the surface of aluminum foil and dried under forced air at 110 °C for 6 h, with the areal density of the active material controlled at 15 mg·cm⁻². A 12 mm positive electrode disc was obtained by cutting.
3. A half cell with positive electrode||separator||lithium metal negative electrode was assembled for charge-discharge testing.
4. Comparative Electrolyte 5 was injected into the battery and allowed to stand at room temperature for 12 h before testing.

### Comparative Example 6

1. As electrolyte solvents, dimethyl carbonate (DMC) and ethylene carbonate (EC) were mixed at a mass ratio of 4:3 to prepare a mixed solvent. NaTFSI and initiator NaPF₆ were dissolved in the above mixed solvent with sufficient dissolution to prepare a base electrolyte containing initiator, wherein the mass fractions of the mixed solvent, NaTFSI and initiator NaPF₆ in the base electrolyte were 69%, 30% and 1%, respectively.
2. The base electrolyte containing initiator in step 1 was added gradually to DOL, with a mass ratio of the base electrolyte to DOL of 1:2. The polymerization precursor solution was stirred at a rotation speed of 600 r·min⁻¹ during mixing, and stirring was continued for 1 h after mixing to prepare a polymerization precursor electrolyte.
3. Na₃V₂(PO₄)₃, conductive carbon and PVDF were mixed at a weight ratio of 90:6:4 and ball-milled for 24 h, with NMP as the solvent and a solid-liquid ratio of 45%. The obtained slurry was coated on the surface of aluminum foil and dried under forced air at 110 °C for 6 h, with the areal density of the active material controlled at 10 mg·cm⁻². A 12 mm positive electrode disc was obtained by cutting.
4. A battery with steel sheet||separator||steel sheet, with blocking electrodes on both sides, was assembled for ionic conductivity testing. A battery with steel sheet||separator||sodium metal, with sodium metal as the reference electrode and counter electrode and steel sheet as the working electrode, was assembled for voltage window testing. A half cell with positive electrode||separator||sodium metal negative electrode was assembled for charge-discharge testing.
5. The polymerization precursor electrolyte was injected into each battery and allowed to stand at room temperature for 24 h to complete curing.

### Comparative Example 7

1. Initiator azobisisobutyronitrile was dissolved in 1,3-dioxolane (DOL) to prepare the first polymerization precursor solution, wherein the mass fraction of the initiator in the first polymerization precursor solution was 0.2%.
2. Non-aqueous organic solvent ethylene carbonate (EC) and ethylene glycol dimethacrylate monomer were mixed at a mass ratio of 5:2 to prepare a mixed solvent. NaFSI and initiator NaODFB were added into the above mixed solvent to prepare the second polymerization precursor solution, wherein the mass fractions of NaFSI and initiator NaODFB in the second polymerization precursor solution were 23% and 0.05%, respectively.
3. The second polymerization precursor solution was added gradually to the first polymerization precursor solution, with a mass ratio of the first polymerization precursor solution to the second polymerization precursor solution of 1:3.5. The polymerization precursor solution was stirred at a rotation speed of 500 r·min⁻¹ during mixing and stirred continuously for 3 h after mixing to prepare a polymerization precursor electrolyte.
4. The methods for electrode preparation and battery assembly and testing were the same as those in Comparative Example 6.
5. The polymerization precursor electrolyte was injected into each battery and allowed to stand at room temperature for 24 h to complete curing.

### Comparative Example 8

1. As electrolyte solvents, dimethyl carbonate (DMC) and ethylene carbonate (EC) were mixed at a mass ratio of 4:3 to prepare a mixed solvent. Sodium salt NaFSI and initiator NaPF₆ were dissolved in the above mixed solvent with sufficient dissolution to prepare a base electrolyte containing initiator, wherein the mass fractions of the mixed solvent, NaFSI and initiator NaPF₆ in the base electrolyte were 69%, 30% and 1%, respectively.
2. The base electrolyte containing initiator in step 1 was added gradually to perfluoro-1,3-dioxolane with a mass ratio of the base electrolyte to perfluoro-1,3-dioxolane of 1:2. The polymerization precursor solution was stirred at a rotation speed of 1200 r·min⁻¹ during mixing, and stirring was continued for 1 h after mixing to prepare a polymerization precursor electrolyte.
3. The methods for electrode preparation and battery assembly were the same as those in Comparative Example 6.
4. The polymerization precursor electrolyte was injected into each battery and allowed to stand at room temperature for 24 h to complete curing.

### Comparative Example 9

1. Initiator azobisisobutyronitrile was dissolved in perfluoro-1,3-dioxolane to prepare the first polymerization precursor solution, wherein the mass fraction of the initiator in the first polymerization precursor solution was 0.2%.
2. Non-aqueous organic solvent ethylene carbonate (EC) and ethylene glycol dimethacrylate monomer were mixed at a mass ratio of 5:2 to prepare a mixed solvent. NaFSI and initiator NaBF₄ were added into the above mixed solvent to prepare the second polymerization precursor solution, wherein the mass fractions of NaFSI and initiator NaBF₄ in the second polymerization precursor solution were 23% and 0.05%, respectively.
3. The second polymerization precursor solution was added gradually to the first polymerization precursor solution, with a mass ratio of the first polymerization precursor solution to the second polymerization precursor solution of 1:3.5. The polymerization precursor solution was stirred at a rotation speed of 600 r·min⁻¹ during mixing and stirred continuously for 2.5 h after mixing to prepare a polymerization precursor electrolyte.
4. The methods for electrode preparation and battery assembly were the same as those in Comparative Example 6.
5. The polymerization precursor electrolyte was injected into each battery and allowed to stand at room temperature for 20 h to complete curing.

### Comparative Example 10

1. As electrolyte solvents, dimethyl carbonate (DMC) and ethylene carbonate (EC) were mixed at a mass ratio of 4:3 to prepare a mixed solvent. Sodium salt NaFSI was dissolved in the above mixed solvent with sufficient dissolution to prepare Comparative Electrolyte 10, wherein the mass fractions of the mixed solvent and NaFSI in the electrolyte were 87% and 13%, respectively.
2. The method for battery preparation was the same as that in Comparative Example 6.
3. A half cell with positive electrode||separator||sodium metal negative electrode was assembled for charge-discharge testing.
4. Comparative Electrolyte 10 was injected into the battery and allowed to stand at room temperature for 12 h before testing.

### Comparative Example 11

1. As electrolyte solvents, dimethyl carbonate (DMC) and ethylene carbonate (EC) were mixed at a mass ratio of 4:3 to prepare a mixed solvent. LiTFSI and initiator LiPF₆ were dissolved in the above mixed solvent with sufficient dissolution to prepare a base electrolyte containing initiator, wherein the mass fractions of the mixed solvent, LiTFSI and initiator LiPF₆ in the base electrolyte were 69%, 30% and 1%, respectively.
2. The base electrolyte containing initiator in step 1 was added gradually to perfluoro-1,3-dioxolane with a mass ratio of the base electrolyte to perfluoro-1,3-dioxolane of 1:2. The polymerization precursor solution was stirred at a rotation speed of 600 r·min⁻¹ during mixing, and stirring was continued for 1 h after mixing to prepare a polymerization precursor electrolyte.
3. The methods for battery preparation and testing were the same as those in Example 20.

### Comparative Example 12

1. As electrolyte solvents, dimethyl carbonate (DMC) and ethylene carbonate (EC) were mixed at a mass ratio of 4:3 to prepare a mixed solvent. Lithium salt LiTFSI was dissolved in the above mixed solvent with sufficient dissolution to prepare Comparative Electrolyte 4, wherein the mass fractions of the mixed solvent and LiTFSI in the electrolyte were 87% and 13%, respectively.
2. The methods for battery preparation and testing were the same as those in Example 20.

The batteries prepared in Examples 1-19 and Comparative Examples 1-10 were subjected to room temperature cycling performance tests under the following conditions.

The prepared batteries were charged to the upper limit of the rated voltage at a constant current of 0.5 C and constant voltage in a constant temperature chamber at an ambient temperature of 25°C, with a cutoff current of 0.02 C. Then, they were discharged at a constant current of 0.5 C until the voltage reached the lower limit of the rated voltage. The cycle was repeated 200 times, and the capacity retention rate was recorded. The capacity retention rate (%) of the nth cycle was calculated as: (specific discharge capacity of the nth cycle / specific discharge capacity of the first cycle) * 100%.

The rated voltage limits are as follows:
LFP batteries: 2.5 V-4.2 V;
NCM622 batteries: 3 V-4.4 V;
NCM811 batteries: 3 V-4.2 V;
LCO batteries: 3 V-4.45 V;
Na₃V₂(PO₄)₃ batteries: 2.5 V-4 V;
Na₃V₂(PO₄)₂F₃ batteries: 2.5 V-4.3 V;
Na₂Fe (SO₄)₂ batteries: 2 V-4.3 V.

The batteries prepared in Examples 20-21 and Comparative Examples 11-12 were fully charged after capacity sorting and subjected to crush and hot box safety tests. The testing requirements were based on GB 38031-2020 Safety Testing Requirements for Power Batteries for Electric Vehicles, with a maximum hot-box test temperature of 150°C.

The test results for Examples 1~11 and Comparative Examples 1~4 are shown in Table 1 below:

**Table 1**

| Group | Oxidation Potential (V) | Ionic Conductivity at Room Temperature (S·cm⁻¹) | Capacity Retention Rate of Batteries (200 cycles) |
|---|---|---|---|
| Example 1 | 5.3 | 5.1×10⁻⁴ | 92.0% |
| Example 2 | 5.4 | 8.9×10⁻⁴ | 92.8% |
| Example 3 | 5.5 | 1.8×10⁻³ | 97.9% |
| Example 4 | 5.2 | 9.0×10⁻⁴ | 97.3% |
| Example 5 | 5.1 | 9.2×10⁻⁴ | 95.4% |
| Example 6 | 5.0 | 6.8×10⁻⁴ | 94.2% |
| Example 7 | 5.1 | 7.3×10⁻⁴ | 97.1% |
| Example 8 | 5.11 | 9.6×10⁻⁴ | 92.4% |
| Example 9 | 5.0 | 8.7×10⁻⁴ | 98.0% |
| Example 10 | 5.4 | 2.2×10⁻³ | 98.3% |
| Example 11 | 5.6 | 8.8×10⁻⁴ | 97.6% |
| Comparative Example 1 | 4.3 | 8.1×10⁻⁵ | 80.2% |
| Comparative Example 2 | 4.6 | 7.8×10⁻⁵ | 81.2% |
| Comparative Example 3 | 4.7 | 7.7×10⁻⁵ | 81.5% |
| Comparative Example 4 | 5.3 | 3.8×10⁻⁴ | 86.3% |

Comparative Example 5 adopts pure liquid electrolyte. Since large deviations exist between ionic conductivity test results obtained by solid electrolyte test methods and conventional test methods, its ionic conductivity was not compared longitudinally. According to tests, the assembled NCM811||Li half-cell maintained a capacity retention rate of 77.8% after 200 cycles at 25 °C.

Test results of Examples 12~19 and Comparative Examples 6~9 are shown in Table 2 below:

**Table 2**

| Group | Oxidation Potential (V) | Ionic Conductivity at Room Temperature (S·cm⁻¹) | Capacity Retention Rate of Batteries (200 cycles) |
|---|---|---|---|
| Example 12 | 4.9 | 5.0×10⁻⁴ | 88.4% |
| Example 13 | 5.1 | 8.4×10⁻⁴ | 90.2% |
| Example 14 | 5.0 | 1.2×10⁻³ | 91.6% |
| Example 15 | 5.2 | 8.7×10⁻⁴ | 90.6% |
| Example 16 | 5.1 | 9.1×10⁻⁴ | 90.2% |
| Example 17 | 5.0 | 6.3×10⁻⁴ | 89.6% |
| Example 18 | 5.1 | 7.0×10⁻⁴ | 89.4% |
| Example 19 | 5.0 | 9.3×10⁻⁴ | 90.8% |
| Comparative Example 6 | 4.4 | 8.0×10⁻⁵ | 79.2% |
| Comparative Example 7 | 4.3 | 7.6×10⁻⁵ | 80.2% |
| Comparative Example 8 | 4.8 | 7.4×10⁻⁵ | 80.5% |
| Comparative Example 9 | 5.0 | 3.6×10⁻⁴ | 84.3% |

Comparative Example 10 adopts pure liquid electrolyte. Since significant deviations exist between ionic conductivity results measured via solid electrolyte testing methods and conventional standard methods, its ionic conductivity was not compared longitudinally. According to test data, the assembled Na₃V₂(PO₄)₃||Na half-cell maintained a capacity retention rate of 75.8% after 200 cycles at 25 °C.

The pouch cell safety test results of Examples 20~21 and Comparative Examples 11~12 are shown in Table 3:

**Table 3**

| Group | Crush Test | Hot Box Test |
|---|---|---|
| Example 20 | No ignition | No obvious swelling |
| Example 21 | No ignition | No obvious swelling |
| Comparative Example 11 | No ignition | Swelling, no ignition |
| Comparative Example 12 | Leakage and ignition | Swelling, leakage and ignition |

From the cycling test results of Examples 1~19, Comparative Examples 1~4, Comparative Examples 6~9, and Comparative Examples 5 and 10, it can be seen that batteries using in-situ solid electrolytes exhibit higher capacity retention rate without additives. Furthermore, the solid electrolytes prepared in Examples 1~10 all show higher oxidation potential, higher ionic conductivity, and better capacity retention rate than Comparative Examples 1~2, indicating that copolymerization of fluoro-1,3-dioxolane compounds with functional groups and free radical polymerization monomers can improve the uniformity of electrolyte polymerization, thereby effectively enhancing electrolyte stability and battery cycling stability. The test results of Examples 12~19 and Comparative Examples 6~9 are similar. Compared with Comparative Examples 3~4 and 8~9, Examples 1~19, in which partial F element in perfluoro-1,3-dioxolane is replaced by functional groups, possess higher ionic conductivity, demonstrating that appropriate substitution of F atoms in perfluoro-1,3-dioxolane facilitates ion transport while retaining the oxidation resistance enhancement brought by F groups. In addition, substitution with specially functional groups-such as cyano and trimethylsiloxy groups-can further improve battery cycling stability and safety. The complexation of cyano groups with transition metal ions protects the positive electrode interface containing transition metals, further boosting battery stability. The excellent stability and migration-blocking effect of trimethylsiloxy groups impart flame-retardant properties, further enhancing the flame retardancy of the electrolyte and improving battery safety. Although perfluoro-1,3-dioxolane shows decent flame retardancy due to its high F element content, its combustion generates a large amount of harmful hydrogen fluoride (HF) gas, which is highly toxic and difficult to recycle. In contrast, silicon-containing compounds achieve flame retardancy by migrating to the material surface during combustion and forming a homogeneous Si-C thermal-insulating char layer after combustion with organic substances, blocking contact between internal unburned organics and external oxygen and inhibiting further combustion, representing a safer flame-retardant mechanism compared with halogen gas-based ones. Therefore, trimethylsiloxy substitution not only retains good flame retardancy but also reduces pollution risks. Meanwhile, F element alone provides relatively weak flame retardancy. Safety tests were carried out by assembling pouch cells. The results show that electrolytes prepared by copolymerization of fluoro-1,3-dioxolane compounds VI and VII with functional groups and free radicals exhibit better safety performance than perfluoro-1,3-dioxolane electrolytes, 1,3-dioxolane electrolytes and pure liquid electrolytes. The safety test results of Examples 20~21 and Comparative Examples 11~12 confirm that fluoro-1,3-dioxolane compounds with special functional groups can further enhance the thermal stability of electrolytes and improve battery safety performance. In addition, vinyl compounds with asymmetric structures have unbalanced electron cloud densities at both ends of the double bond, which is unfavorable for cyclization into fluoro-1,3-dioxolane compounds. Therefore, all vinyl compounds used in this disclosure have symmetric structures.

The present invention provides an in-situ solid electrolyte obtained by curing a mixture comprising a fluoro-1,3-dioxolane compound, a free radical polymerization monomer, a first initiator, a second initiator, an electrolyte salt and a non-aqueous organic solvent, wherein the fluoro-1,3-dioxolane compound is selected from any one of Formulas I to X. In the present invention, a first polymerization precursor solution of the first initiator and the fluoro-1,3-dioxolane compound, and a second polymerization precursor solution containing the electrolyte salt, the second initiator and the free radical polymerization monomer are prepared separately. After mixing the first and the second polymerization precursor solutions, the second initiator initiates polymerization of the fluoro-1,3-dioxolane compound, and heat released during this process promotes the first initiator to trigger polymerization of the free radical polymerization monomer. The free radical polymerization features fast chain growth and tends to form long chains to construct a free radical polymer network, while polymerization of the fluoro-1,3-dioxolane compound exhibits slow chain growth and tends to form short chains. Therefore, the free radical polymer network crosslinks short chains of polyfluoro-1,3-dioxolane with high chain mobility, contributing to high ionic conductivity. In the preparation method for an in-situ solid electrolyte provided by the present invention, by means of the exothermic ionic polymerization of fluoro-1,3-dioxolane compound at room temperature, free radical comonomer polymerization is initiated, avoiding non-uniform polymerization caused by external heating. In addition, the free radical comonomer polymerization can consume heat released by ion polymerization, resulting in a milder curing process without bubble residues, so that the in-situ solid electrolyte is more uniform in polymerization, and has better contact with an electrode. In addition, the fluoro-1,3-dioxolane compound can be grafted with various functional groups to modify physical and chemical properties of the solid electrolyte.

The present invention further provides a preparation method for fluoro-1,3-dioxolane compound, comprising mixing a vinyl compound solution and CO₂F₂ at -50 °C~-25 °C under a protective atmosphere, allowing an addition reaction to proceed under the catalysis of a foamed metal, removing the solvent, and purifying the product to obtain the fluoro-1,3-dioxolane compound. Compared with conventional technologies, the present invention eliminates the need for ultra-low temperature reactions such as -80°C, leading to milder reaction conditions. Moreover, the yield of the resulting fluoro-1,3-dioxolane heterocyclic compound is high, reaching above 90%. Fluoro groups in the present invention possess excellent chemical stability, enhancing stability of the solid electrolyte. The fluoro-1,3-dioxolane compound can also be grafted with functional groups to optimize physical and chemical properties of the solid electrolyte, and improve safety performance, electrochemical stability and energy density thereof.

Researches show that the in-situ solid electrolyte prepared by the above method has a wide electrochemical window with an oxidation voltage higher than 4.5 V, suitable for high-voltage lithium battery and high-voltage sodium battery systems, and its ionic conductivity is higher than 5×10⁻⁴ S/cm. When applied in secondary batteries, taking a lithium ion half-cell as an example, the cycling capacity retention rate (after 100 cycles) of the lithium ion half-cell is above 90%.

### INDUSTRIAL APPLICABILITY

The present invention provides a preparation method for a fluoro-1,3-dioxolane compound, an in-situ solid electrolyte, and a preparation method therefor and a use thereof. The fluoro-1,3-dioxolane compounds prepared by the disclosed method can be added into solid electrolytes to improve safety performance, electrochemical stability and energy density of solid electrolytes. The in-situ solid electrolyte of the present invention achieves high ionic conductivity and a wide electrochemical window, and is suitable for high-voltage battery systems.

## Claims

1. An in-situ solid electrolyte, **characterized in that** the electrolyte is obtained by polymerizing a mixture, wherein
the mixture comprises a fluoro-1,3-dioxolane compound, a free radical polymerization monomer, a first initiator, a second initiator, an electrolyte salt and a non-aqueous organic solvent;
the fluoro-1,3-dioxolane compound is any one selected from the group consisting of Formulas I to X below:

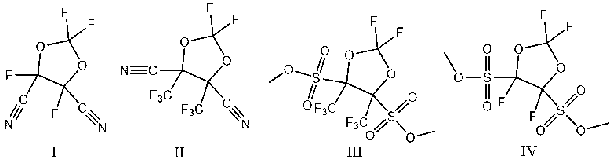

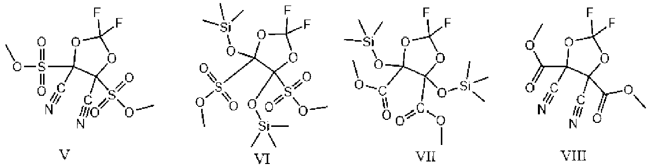

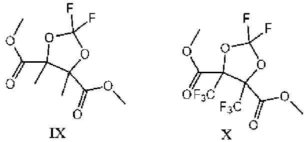

2. The in-situ solid electrolyte according to claim 1, wherein the free radical polymerization monomer is any one or more selected from the group consisting of vinyl ethylene carbonate, methyl vinyl sulfone, ethyl vinyl sulfone, triethylene glycol divinyl ether, methyl methacrylate, vinyl acetate, 1,3-propene sultone, acrylamide, ethyl methacrylate, n-butyl methacrylate, vinylene carbonate, maleic anhydride, succinonitrile, trimethylolpropane triacrylate, ethylene glycol dimethacrylate, vinyl acetate, polyethylene glycol diacrylate and n-butyl acrylate;
the first initiator is selected from azo initiators or peroxide initiators;
the second initiator is any one or more selected from the group consisting of lithium hexafluorophosphate, lithium tetrafluoroborate, lithium difluoro(oxalato)borate, sodium hexafluorophosphate, sodium tetrafluoroborate, sodium difluoro(oxalato)borate, boron trifluoride, niobium pentachloride, titanium tetrachloride, aluminum chloride, ferric chloride and aluminum trifluoromethanesulfonate;
the electrolyte salt comprises lithium salt and sodium salt;
the lithium salt is any one or more selected from the group consisting of LiPF₆, LiClO₄, LiBF₄, LiPOF₂, LiTFSI, LiFSI, LiODFB and LiBOB;
the sodium salt is any one or more selected from the group consisting of NaPF₆, NaClO₄, NaBF₄, NaTFSI, NaFSI and NaODFB; and
the non-aqueous organic solvent is any one or more selected from the group consisting of ethylene carbonate, propylene carbonate, diethyl carbonate, ethyl methyl carbonate, dimethyl carbonate and ethylene glycol dimethyl ether.

3. A preparation method for the in-situ solid electrolyte according to claim 1 or 2, **characterized in that** the method comprises following steps:
S1: mixing the first initiator and the fluoro-1,3-dioxolane compound shown in any one of Formulas I to X to obtain a first polymerization precursor solution; mixing the electrolyte salt, the second initiator, the free radical polymerization monomer and the non-aqueous organic solvent to obtain a second polymerization precursor solution; and
S2: mixing the first polymerization precursor solution and the second polymerization precursor solution to obtain a polymerization precursor electrolyte, and injecting the polymerization precursor electrolyte into a battery containing a positive electrode active material for curing to obtain the in-situ solid electrolyte.

4. The preparation method for the in-situ solid electrolyte according to claim 3, wherein in step S1, a mass ratio of the non-aqueous organic solvent to the free radical polymerization monomer is 5: (1~10);
a mass fraction of the first initiator in the first polymerization precursor solution is 0.01%~5%;
a mass fraction of the second initiator in the second polymerization precursor solution is 0.01%~3%; and
a mass fraction of the electrolyte salt in the second polymerization precursor solution is 12%~30%.

5. The preparation method for the in-situ solid electrolyte according to claim 3, wherein in step S2, a mass ratio of the first polymerization precursor solution to the second polymerization precursor solution is 1: (1~5); and
the polymerization is room-temperature polymerization, and a duration of the polymerization is 12 h~72 h.

6. Use of the in-situ solid electrolyte according to claim 1 or 2 or the in-situ solid electrolyte prepared by the preparation method for the in-situ solid electrolyte according to any one of claims 3 to 5 in a solid-state secondary battery, **characterized in that** the solid-state secondary battery comprises a positive electrode, a negative electrode, a separator and an electrolyte, wherein
the electrolyte is the in-situ solid electrolyte according to claim 1 or 2 or the in-situ solid electrolyte prepared by the preparation method for the in-situ solid electrolyte according to any one of claims 3 to 5.

7. The use according to claim 6, wherein the solid-state secondary battery is a solid-state lithium-ion battery or a solid-state sodium-ion battery.

8. A preparation method for a fluoro-1,3-dioxolane compound, **characterized in that** the method comprises following steps:
mixing a vinyl compound solution and CO₂F₂ at -50 °C~-25 °C under a protective atmosphere, allowing an addition reaction to proceed under a catalysis of a foamed metal, removing a solvent, and purifying a product to obtain the fluoro-1,3-dioxolane compound.

9. The preparation method according to claim 8, wherein the protective atmosphere is argon; and
the foamed metal is any one or more selected from the group consisting of foamed iron, foamed nickel and foamed copper.

10. The preparation method according to claim 8, wherein a molar ratio of a vinyl compound in the vinyl compound solution to CO₂F₂ is 1: (1~5).

11. The preparation method for the fluoro-1,3-dioxolane compound according to claim 8, wherein a temperature of the addition reaction is -50 °C~-25 °C, and a duration of the addition reaction is 6 h~48 h.

12. The preparation method for the fluoro-1,3-dioxolane compound according to claim 8, wherein the vinyl compound solution is obtained by mixing a vinyl compound and the solvent at -50 °C~-25 °C under the protective atmosphere, followed by heating to room temperature; and
the solvent in the vinyl compound solution is any one or more selected from the group consisting of acetonitrile, dichloromethane, trichloromethane, dichloroethane, propanol, acetone, dioxane, tetrahydrofuran, methyl ethyl ketone, n-butanol, ethyl acetate, diethyl ether and chloroform.

13. The preparation method for the fluoro-1,3-dioxolane compound according to claim 8, wherein a vinyl compound in the vinyl compound solution is any one selected from the group consisting of Formula A to Formula J:

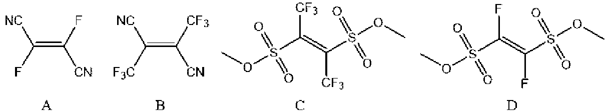

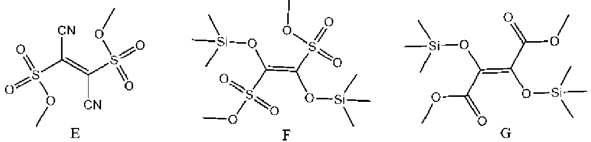

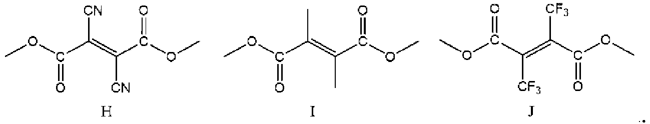

14. The preparation method for the fluoro-1,3-dioxolane compound according to claim 12, wherein a molar ratio of the vinyl compound to the solvent is 1: (2~10); and
a heating rate is 0.1 °C/min~5 °C/min.

15. The preparation method for the fluoro-1,3-dioxolane compound according to claim 8, wherein after the addition reaction is completed, the resultant is heated to room temperature under the protective atmosphere, and then the solvent is removed to obtain the fluoro-1,3-dioxolane compound.
